# EUROPEAN PATENT APPLICATION

(11) **EP 2 068 152 A1**
(43) Date of publication of application: **10.06.2009**
(21) Application number: 07023667.4
(22) Date of filing: 06.12.2007
(51) Int. Cl.: G01N 33/94, G01N 33/50, C12Q 1/48, A61K 31/44

(54) **c-Kit as a novel target for the treatment of pain**

(71) Applicant: Max-Delbrück-Centrum für Molekulare Medizin (MDC), 13092 Berlin (DE)
(72) Inventor: Lewin, Garry R., 13158 Berlin (DE)
(74) Representative: Krauss, Jan

(57) **Abstract**

The enhanced sensitivity we feel following injury is called hyperalgesia. In this invention a receptor that is a known player in human cancer, called c-Kit, was examined in the context of pain and hyperalgesia, and used for the development of compounds and medicaments that are effective against pain and hyperalgesia. The c-Kit receptor is expressed on pain sensing sensory neurons and mice lacking the receptor have reduced sensitivity to painful heating of the skin.

## Description

The present invention relates to the tyrosine kinase c-Kit as a novel target for the treatment of pain. The invention provides screening and therapeutic methods utilizing the tyrosine kinase c-Kit as well as pharmaceutical preparations based on the results of said screening. The following description of the background of the invention is provided to aid in understanding the invention, but is not admitted to describe or constitute prior art to the invention.

### Background of the invention

The enhanced sensitivity we feel following injury is called hyperalgesia. In this invention a receptor that is a known player in human cancer, called c-Kit, was examined in the context of pain and hyperalgesia, and used for the development of compounds and medicaments that are effective against pain and hyperalgesia. The c-Kit receptor is expressed on pain sensing sensory neurons and mice lacking the receptor have reduced sensitivity to painful heating of the skin.

Somatic sensations such as warming, cooling, gentle touch and pain are each initiated by activation of sensory neurons. Specific types of sensory neurons, whose cell bodies are located in dorsal root and trigeminal ganglia, subserve different sensory modalities. Specialized sensory neurons called nociceptors are responsible for the transduction of painful thermal and mechanical stimulation of the skin. Knowledge about molecules and ion channels that are necessary for the normal transduction of painful thermal and mechanical stimuli is still incomplete (Lewin and Moshourab, 2004). It has been postulated that thermosensitive ion channels of the TRP family are important for the transduction of noxious heat or cold by nociceptive sensory neurons (Jordt et al., 2003). The most complete evidence exists for the capsaicin activated ion channel TRPV1 that can be activated by thermal stimuli in the noxious range (Caterina et al., 1997; Tominaga et al., 1998). Mice lacking TRPV1 have altered pain behavior and do not respond to the noxious irritant capsaicin (Caterina et al., 2000; Davis et al., 2000).

An important feature of pain is the fact that injury and inflammation leads to heightened sensitivity to stimuli that would normally be only mildly painful. This phenomenon is called hyperalgesia, and the prevention of hyperalgesia is a hallmark of effective analgesia. TRPV1 may become an important analgesic target because this channel is required for the expression of thermal hyperalgesia provoked by inflammation (Caterina et al., 2000; Davis et al., 2000).

Molecules up-regulated in inflamed tissue such as nerve growth factor (NGF) can sensitize peripheral nociceptors to thermal stimuli (Lewin et al., 1993). NGF signaling via its receptor tyrosine kinase TrkA is necessary for the survival of sub-populations of peripheral neurons during periods of programmed cell death (Huang and Reichardt, 2003).

It has been known for many years that the dorsal root ganglion (DRG) neurons that require NGF are all nociceptors (Lewin and Mendell, 1993). NGF/TrkA signaling is required for neuronal survival during embryonic development, but in mature animals NGF is also a physiological mediator of inflammatory hyperalgesia (Lewin et al., 1994; Woolf et al., 1994). Thus, NGF can produce a profound and long lasting thermal and mechanical hyperalgesia in man and animals (Lewin et al., 1993; Petty et al., 1994). NGF can also potentiate TRPV1 mediated and noxious heat activated ionic currents (Iₕₑₐₜ) in isolated DRG neurons (Galoyan et al., 2003; Shu and Mendell, 1999). Indeed, NGF injected into animals produces thermal hyperalgesia that requires the presence of TRPV1 (Chuang et al., 2001).

Around half of the nociceptors in the adult DRG possess TrkA receptors; the remainder, defined by the expression of c-Ret, downregulate TrkA during early postnatal development (Luo et al., 2007; Snider and McMahon, 1998). The receptor tyrosine kinase c-Ret mediates signals elicited by the glial-derived neurotophic factor (GDNF) ligand family (Airaksinen and Saarma, 2002). The c-Ret receptor and its co-receptors GFRα2 and 3 are present in nociceptive neurons, some of which are heat sensitive and express TRPV1 receptors (Malin et al., 2006; Stucky et al., 2002). Indeed, there is some evidence for a role of the GDNF family ligands neurturin and artemin in regulating noxious heat transduction by sensory neurons (Malin et al., 2006; Stucky et al., 2002). GDNF signaling is however not required for survival of nociceptive sensory neurons (Airaksinen and Saarma, 2002; Luo et al., 2007).

In addition to the Trk and c-Ret receptors, sensory neurons are known to express other receptor tyrosine kinases like c-Kit, the receptor for stem cell factor (SCF) (Hirata et al., 1993; Keshet et al., 1991; Motro et al., 1991). The c-Kit/SCF signaling system has interested geneticists for almost a century: allelic mutations in SCF (*Steel, Sl*) or c-Kit (*W*) give rise to dominant white spotting pigment phenotypes (Little, 1915), and their analysis provided the first genetic evidence for an essential function of receptor tyrosine kinase signaling in mammalian development (Besmer, 1991).

SCF/c-Kit signaling has diverse functions in the development of primordial germ cells, melanocytes, and haematopoietic lineages (mast cells and erythroid progenitors) (Besmer, 1991). The embryonic or perinatal lethality in mutants homozygous for loss-of function *Sl* or *W* alleles has hampered the study of c-Kit/SCF signaling functions in the adult (Besmer, 1991; Reith et al., 1990). Nevertheless, the availability of inbred W mutant substrains, originally established during intensive inbreeding regimens to select for postnatal survival of homozygote mutants (Russell and Lawson, 1959), as well as transgenic approaches that rescue the haematopoietic defect in *c-Kit* mutants permit the generation of adult mice completely lacking c-Kit function (Waskow et al., 2002). However, nervous system phenotypes have not been reported in such animals.

Deininger et al (in: Deininger M, Buchdunger E, Druker BJ. The development of imatinib as a therapeutic agent for chronic myeloid leukemia. Blood. 2005 Apr 1;105(7):2640-53.) describe Imatinib as drug therapy of chronic myeloid leukemia (CML) and review the preclinical and clinical development of imatinib for the therapy of CML, resistance and strategies that may help to eliminate resistant or residual leukemia.

US 7,211,600 relates to methods, compounds, and compositions for inhibiting cell proliferative disorders characterized by overactivity and/or inappropriate activity of a c-kit kinase.

US 5,625,121 relates to a mouse homozygous for a disrupted trkB gene, wherein the trkB gene is disrupted by the insertion of a selectable marker sequence and wherein said mouse exhibits a decrease in the number of neurons which comprise the facial motor nucleus, spinal cord, trigeminal ganglia and dorsal root ganglia.

As mentioned above, the treatment and/or prevention of hyperalgesia, in particular in the context of cancer, are hallmarks of effective analgesia. Therefore, pharmaceutically active compounds are sought for that have an effect on the biological components and pathways involved in hyperalgesia. Further sought for are methods to identify said compounds.

According to a first aspect of the present invention, one object of the invention is solved trough providing a method for identifying a compound that modulates the expression and/or activity of the tyrosine kinase receptor c-Kit in a cell, comprising the steps of a) contacting a cell expressing the tyrosine kinase receptor c-Kit with at least one potentially modulating compound, and b) measuring the modulation of the expression and/or activity of the tyrosine kinase receptor c-Kit in said cell.

In the context of the present invention, the inventors defined key functions of the receptor tyrosine kinase c-Kit and its ligand SCF in peripheral pain and mechanosensation, using genetic and electrophysiological approaches. The inventors' analysis revealed two distinctive roles for c-Kit signaling, namely:
(i) c-Kit provides a key input to regulate the heat sensitivity of nociceptors;
(ii) c-Kit signaling regulates the function of discrete types of mechanosensitive sensory neurons and loss of c-Kit leads to mechanoreceptor hypersensitivity.
   Thus the finding that c-Kit is an essential regulator of nociceptor sensitivity identified it as a new target for pain therapy.

Another important aspect of the present invention relates to a pharmaceutical composition for treating or preventing pain, obtainable by a method according to the present invention, as described herein.

Yet another important aspect of the present invention relates to a method for treating or preventing pain, comprising administering to a subject in need thereof an effective amount of a pharmaceutical composition according to the present invention as above.

Yet another important aspect of the present invention relates to the use of a compound as identified according to a method according to the present invention, or the pharmaceutical composition according to the present invention for treating or preventing a disorder selected from pain, hyperalgesia, thermal hyperalgesia, cancer pain, and inflammatory pain.

Yet another important aspect of the present invention relates to the use of imatinib (4-[(4-Methyl-1-piperazinyl)methyl]-N-[4-methyl-3-[[4-(3-pyridinyl)-2-pyrimidinyl]amino]-phenyl]benzamide) for the production of a medicament for the treatment of a disorder selected from pain, hyperalgesia, thermal hyperalgesia, cancer pain, and inflammatory pain.

The molecular mechanisms regulating the sensitivity of sensory circuits to environmental stimuli are poorly understood. The inventors demonstrate here a central role for Stem Cell Factor (SCF, as an example) and its receptor, c-Kit, in tuning the responsiveness of sensory neurons to natural stimuli. Mice lacking SCF/c-Kit signaling displayed profound thermal hypoalgesia, attributable to a marked elevation in the thermal threshold and reduction in spiking rate of heat-sensitive nociceptors. Acute activation of c-Kit by its ligand, SCF, resulted in a reduced thermal threshold and potentiation of heat-activated currents in isolated small diameter neurons, and thermal hyperalgesia in mice. SCF induced thermal hyperalgesia required the TRP-family cation channel TRPV1. Lack of c-Kit signaling during development resulted in hypersensitivity of discrete mechanoreceptive neuronal subtypes. Thus, c-Kit can now be grouped with a small family of receptor tyrosine kinases, including c-Ret and TrkA, that control the transduction properties of sensory neurons.

The method for identifying a compound that modulates the expression and/or activity of the tyrosine kinase receptor c-Kit in a cell according to the invention is suitable for the determination of compounds that can interact with the proteins of the present invention and to identify, for example, inhibitors, activators, competitors or modulators of proteins of the present invention, in particular inhibitors, activators, competitors or modulators of the enzymatic activity of the proteins of the present invention. Preferred is a method according to the invention wherein said compound as identified is an inhibitor or activator of the expression and/or biological activity of the tyrosine kinase receptor c-Kit in said cell. Most preferred is an inhibitor.

The potentially binding substance, whose binding to the tyrosine kinase receptor c-Kit is to be measured, can be any chemical substance or any mixture thereof. For example, it can be a substance of a peptide library, a nucleic acid library, a combinatory library, a cell extract, in particular a plant cell extract, a "small molecular drug", a protein and/or a protein fragment.

The term "contacting" in the present invention means any interaction between the potentially binding substance(s) with the tyrosine kinase receptor c-Kit, whereby any of the two components can be independently of each other in a liquid phase, for example in solution, or in suspension or can be bound to a solid phase, for example, in the form of an essentially planar surface or in the form of particles, pearls or the like. In a preferred embodiment a multitude of different potentially binding substances are immobilized on a solid surface like, for example, on a compound library chip and the protein of the present invention is subsequently contacted with such a chip.

The tyrosine kinase receptor c-Kit employed in a method of the present invention can be full length proteins or a fragments with N/C-terminal and/or internal deletions. Preferably the fragments are either N-terminal fragments comprising the enzymatic region of the protein or C-terminal fragments comprising the cytoplasmic region, depending on whether potentially interacting compounds are sought that specifically interact with the N- or C-terminal fragment.

Measuring of binding of the compound to the tyrosine kinase receptor c-Kit can be carried out either by measuring a marker that can be attached either to the protein or to the potentially interacting compound. Suitable markers are known to someone of skill in the art and comprise, for example, fluorescence or radioactive markers. The binding of the two components can, however, also be measured by the change of an electrochemical parameter of the binding compound or of the protein, e.g. a change of the redox properties of either the protein or the binding compound, upon binding. Suitable methods of detecting such changes comprise, for example, potentiometric methods. Further methods for detecting and/or measuring the binding of the two components to each other are known in the art and can without limitation also be used to measure the binding of the potential interacting compound to the tyrosine kinase receptor c-Kit or fragments thereof. The effect of the binding of the compound or the activity of the tyrosine kinase receptor c-Kit can also be measured indirectly, for example, by assaying the kinase activity of the protein after binding.

In some instances it might be desirable to interfere with, for example, the transcription or translation of the tyrosine kinase receptor c-Kit and, therefore, the present invention is also directed at a nucleic acid, which is complementary to the nucleic acid encoding the tyrosine kinase receptor c-Kit and, thus, is capable of inhibiting, for example, transcription or translation. A preferred embodiment of such a complementary nucleic acid is a so called anti-sense oligonucleotide (R. Q. Zheng and D. M. Kemeny (1995) Clin. Exp. Immunol. 100:380-2, W. Nellen and C. Lichtenstein (1993) Trends. Biochem. Sci. 18:419-423 and C. A. Stein (1992) Leukemia 6:967-74), ribozymes (M. Amarzguioui and H. Prydz (1998) Cell. Mol. Life Sci. 54:1175-1202, N. K. Vaish et al (1998) Nucleic Acids Res. 96:5237-5242, Persidis (1997) Nat. Biotechnol. 15:921-922 and L. A. Couture and D. T. Stinchcomb (1996) Trends Genet. 12:510-515) and/or so called small interfering RNA-molecules (siRNAs or RNAi's) (S. M. Elbashir et al. (2001) Nature 411:494-498, Rondinone CM. RNAi for the Identification of New Targets for the Treatment of Metabolic Diseases. Endocrinology. 2006 Mar 23). Antisense oligonucleotides are able to decrease the stability of the above described nucleic acids and/or can inhibit the translation. Similarly the use of siRNA-oligonucleotides can also lead to a reduction in the amount of the translated polypeptides. Anti-sense oligonucleotides have in a preferred embodiment a length of at least 20, preferable of at least about 30, more preferably of at least about 40 and most preferably a length of at least about 50 nucleic acids.

Oligonucleotides are generally rapidly degraded by endo- or exonucleases, which are present in the cell, in particular by DNases und RNases and, therefore, it is advantageous to modify the nucleic acids which are used, for example, in anti-sense strategies, as ribozymes or siRNAs to stabilize them against degradation and thereby prolong the time over which an effective amount of the nucleic acid is maintained within the cell (L. Beigelmann et al. (1995) Nucleic acids Res. 23:3989-94, WO 95/11910, WO 98/37340 and WO 97/29116). Typically such stabilization can be obtained by the introduction of one or more internucleotide phosphate groups and/or by the introduction of one or more non-phosphor-internucleotides.

Suitable modified internucleotides are summarized in, for example, Uhlmann and Peimann (1990) Can. Rev. 90:544. Modified internucleotide phosphate residues and/or non-phosphate bridges which can be used in a nucleic acid of the invention comprise, for example, methylphosphonate, phosphorthioate, phosphoramidate, phosphordithionate, phosphate ester, non-phosphor internucleotide analogues, which can be used in nucleic acids of the invention include, for example, siloxane bridges, carbonate bridges, carboxymethylester, acetamid bridges and/or thioether bridges.

As a further step after measuring the binding of a potentially interacting compound and after having measured at least two different potentially interacting compounds at least one compound can be selected, for example, on grounds of the measured binding activity or on grounds of the detected increase or decrease of protein activity, in particular kinase activity upon binding. Preferred is a method according to the invention, wherein said method is performed in vivo in a non-human animal or in vitro. Further preferred is a method according to the invention, wherein said cell is selected from neuronal cells, cells recombinantly expressing the tyrosine kinase receptor c-Kit, and cells in non-human animals, or organisms. The term "organism" relates to any living entity comprised of at least one cell. An organism can be as simple as one eukaryotic cell or as complex as a mammal. The organism is preferably a mammal, more preferably a human. The term "mammal" refers preferably to such organisms as mice, rats, rabbits, guinea pigs, sheep, and goats, more preferably to cats, dogs, monkeys, and apes.

Preferred is a method according to the invention, wherein said activity of the tyrosine kinase receptor c-Kit is selected from the inhibition of the binding of stem cell factor to c-Kit, tyrosine kinase phosphorylation, and/or down-regulating pain transduction by sensory neurons of the dorsal root ganglion (DRG). The term "activity" used above, in the context of the invention, also defines the rate at which a protein kinase phosphorylates a substrate. Catalytic activity can be measured, for example, by determining the amount of a substrate converted to a product as a function of time. Phosphorylation of a substrate occurs at the active site of a protein kinase. The active site is normally a cavity in which the substrate binds to the protein kinase and is phosphorylated. The term "substrate" as used herein refers to a molecule phosphorylated by a protein kinase. The substrate is preferably a peptide and more preferably a protein. The term "function" as used in relation to a protein kinase above refers to the cellular role of a c-kit kinase. The protein kinase family includes members that regulate many steps in signaling cascades, including cascades controlling cell growth, migration, differentiation, gene expression, muscle contraction, glucose metabolism, cellular protein synthesis, and regulation of the cell cycle. The "function" of a membrane receptor kinase usually is to transduce a signal from outside a cell's membrane to the interior of a cell. To accomplish this it may perform one or all of these other functions: bind a ligand, dimerize to another membrane receptor kinase, phosphorylate other proteins within the cell, bind other proteins within the cell, and cause the localization of proteins within the cell.

Preferred is a method according to the invention as above, wherein said identifying further comprises identifying a pharmaceutically active compound suitable for treating or preventing a disorder selected from pain, hyperalgesia, thermal hyperalgesia, cancer pain, and inflammatory pain.

Particularly preferred is a method according to the invention, wherein said identifying further comprises identifying a pharmaceutically active compound that exhibits a dual effect as anti-cancer compound and analgesic compound, i.e. has a therapeutic effect on both abnormal conditions.

Preferred is a method according to the invention, wherein said compound as identified is selected from a peptide library, a combinatory library, a cell extract, in particular a plant cell extract, a "small molecule drug", an antisense oligonucleotide, an siRNA, an mRNA or nucleic acid encoding c-Kit or a mutated form thereof, and an antibody or fragment thereof specifically recognizing the amino acid sequence of c-Kit, in particular the small molecule drug imatinib (4-[(4-Methyl-1-piperazinyl)methyl]-N-[4-methyl-3-[[4-(3-pyridinyl)-2-pyrimidinyl]amino]-phenyl]benzamide).

The thus selected binding compound is than in a preferred embodiment modified in a further step. Modification can be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or iso-pentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

The thus modified binding substances are than individually tested with the method of the present invention, i.e. they are contacted with the protein and subsequently binding of the modified compounds to the protein is measured. In this step both the binding per se can be measured and/or the effect of the function of the protein like, e.g. the enzymatic activity of the protein can be measured. If needed the steps of selecting the binding compound, modifying the binding compound, contacting the binding compound with a protein of the invention and measuring the binding of the modified compounds to the protein can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity, in particular the homeobox transcription factor activity of the proteins of the present invention.

Another aspect of the present invention then relates to a method for manufacturing a pharmaceutical composition for treating or preventing pain, comprising the steps of: performing a method according to the invention as above, and formulating said compound as identified into a pharmaceutical composition.

In a further embodiment of the method of the present invention the interacting compound identified as outlined above or the functional interactor identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or the functional interactor or comprise substances or materials, which have to be included for certain routs of application like, for example, intravenous solution, sprays, Band-Aids or pills.

Preferred is a method according to the invention, wherein said pharmaceutical composition is for oral administration.

Another aspect of the present invention then relates to a pharmaceutical composition for treating or preventing pain, obtainable by a method according to the invention as described herein.

Yet another aspect of the present invention then relates to a method for treating or preventing pain, comprising administering to a subject in need thereof an effective amount of a pharmaceutical composition according to the present invention. Thus, in another aspect, the invention provides a method for treating or preventing an abnormal condition in an organism. The abnormal condition is associated with an aberration in a signal transduction pathway mediated by an interaction between a c-kit kinase and a natural binding partner. The method involves administering to the organism a therapeutically effective amount of a compound as identified according to the present invention. The compound modulates the interaction between the c-kit kinase and a natural binding partner. Therefore, promoting or disrupting (preferably disrupting) this interaction is predicted to have therapeutic benefits to a given population of patients in need of such treatment. In a preferred embodiment, the amount of signaling through c-kit kinase is abnormal, and the compound promotes or disrupts the signaling.

The term "treating" refers to having a therapeutic effect and at least partially alleviating or abrogating an abnormal condition in the organism. The term "treating" preferably refers to ameliorating a symptom of the abnormal condition in a group of patients to whom the compound is administered relative to a control group that does not receive the compound. The effect of the treatment can be monitored by measuring a change or an absence of a change in cell phenotype, a change or an absence of a change in the catalytic activity of this c-kit protein kinase, and a change or an absence of a change in the interaction between this protein kinase and a natural binding partner. The term "treating" or "treatment" does not necessarily mean total cure. Any alleviation of any undesired symptom of the disease to any extent can be considered treatment. Furthermore, treatment may include acts which may worsen the patient's overall feeling of well being or appearance. A "therapeutically effective" amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein. A "therapeutically effective amount," in reference to the treatment of a cancer refers to an amount sufficient to bring about one or more of the following results: reduce the size of the cancer, inhibit the metastasis of the cancer, inhibit the growth of the cancer, stop the growth of the cancer, relieve discomfort due to the cancer, or prolong the life of a patient inflicted with the cancer. A "therapeutically effective amount", in reference to the treatment of pain refers to an amount sufficient to bring about one or more of the following results: relieve discomfort due to the disorder, or prolong the life of a patient suffering from the disorder.

The term "abnormal condition" refers to a function in the cells or tissues of an organism that deviates from its normal functions in that organism. An abnormal condition can relate to cell proliferation, cell differentiation, or cell survival. Abnormal conditions include asthma, allergy-associated chronic rhinitis, small cell lung cancer, non-small cell lung cancer, acute myelocytic leukemia, acute lymphocytic leukemia, myelodysplastic syndrome, chronic myelogenous leukemia, colorectal carcinomas, gastric carcinomas, gastrointestinal stromal tumors, testicular cancers, glioblastomas, and astrocytomas. In a preferred embodiment, this abnormal conditions, such as pain, arises in non-human organisms and may thus be prevented or treated during the practice of medicine.

Yet another aspect of the present invention then relates to the use of a compound as identified according to a method according to the present invention, or the pharmaceutical composition according to the present invention for treating or preventing a disorder selected from pain, hyperalgesia, thermal hyperalgesia, cancer pain, and inflammatory pain.

Yet another aspect of the present invention then relates to the use of imatinib (4-[(4-Methyl-1-piperazinyl)methyl]-N-[4-methyl-3-[[4-(3-pyridinyl)-2-pyrimidinyl]amino]-phenyl]benzamide) for the production of a medicament for the treatment of a disorder selected from pain, hyperalgesia, thermal hyperalgesia, cancer pain, and inflammatory pain.

In view of the above, the present invention is furthermore directed in part towards compounds and methods of modulating the function of the protein kinase c-Kit with these compounds. In addition, the invention describes methods of treating and preventing protein kinase-related abnormal conditions, such as pain, in organisms with a compound identified by the methods described herein. Furthermore, the invention pertains to pharmaceutical compositions containing compounds identified by methods of the invention.

The present invention features compounds that potently inhibit the receptor protein kinase c-kit family and related products and methods. Other inhibitors and/or activators of c-kit protein kinase can be obtained by adding chemical substituents to an unsubstituted compound as identified as above. The compounds of the invention provide therapeutics and/or prophylactics for conditions associated with functional c-kit protein kinase, such as pain and cancer-related pain. Certain types of cancers fall into this class of diseases, along with certain immune disorders associated with the over-production or over-stimulation of mast cells. The compounds can be modified such that they are specific or more specific (i.e. have enhanced kinetics for the binding) to their c-kit target or targets, and will subsequently cause few side effects. These properties are significant improvements over some of the currently utilized pain therapeutics that cause multiple side effects and deleteriously weaken patients. Certain types of cancer, such as Small Cell Lung Cancer (SCLC), express both the c-kit receptor protein kinase and Stem Cell Factor (SCF), a c-kit ligand.

While a precise understanding of the mechanism by which compounds inhibit phosphotyrosine kinases (PTKs) (e.g., the c-kit receptor kinase, a transmembrane tyrosine kinase growth factor receptor) is not required in order to practice the present invention, the compounds are believed to interact with the amino acids of the PTKs' catalytic region. PTKs typically possess a bi-lobate structure, and ATP appears to bind in the cleft between the two lobes in a region where the amino acids are conserved among PTKs; inhibitors of PTKs are believed to bind to the PTKs through non-covalent interactions such as hydrogen bonding, Van der Waals interactions, hydrophobic interactions, and ionic bonding, in the same general region that ATP binds to the PTKs. Specificity of a PTK inhibitor for a particular PTK may be conferred by interactions between the constituents around the oxindole core with amino acid domains specific to individual PKs. Thus, different substitutents may contribute to preferential binding to particular PKs. The ability to select those compounds active at different ATP binding sites makes them useful in targeting any protein with such a site, including not only protein tyrosine kinases, but also serine/threonine kinases. Thus, such compounds may have utility for in vitro assays on such proteins and for in vivo therapeutic effect through such proteins.

The term "c-kit kinase" refers to a membrane receptor protein tyrosine kinase which is preferably activated upon binding Stem Cell Factor (SCF) to its extracellular domain (Yarden et al., 1987; Qiu et al., 1988). The receptor tyrosine kinase c-kit kinase contains 5 immunoglobulin-like motifs in the extracellular domain and a cytoplasmic "split" kinase domain. The full length amino acid sequence of a c-kit kinase preferably is as set forth in Yarden, et al., 1987, EMBO J. 11:3341 3351; and Qiu, et al., 1988, EMBO J. 7:1003 1011, which are incorporated by reference herein in their entirety, including any drawings.

The characterization of molecules that regulate stimulus detection and transduction properties of nociceptive sensory neurons is of fundamental importance, since analgesic therapies target the sensitization of nociceptive pathways. The present invention assigns key functions to the receptor tyrosine kinase c-Kit and its natural ligand SCF in the biology of peripheral sensory neurons. The inventors show that signaling through c-Kit acutely tunes the sensitivity and heat threshold of polymodal nociceptors. Furthermore, c-Kit mediated signals influence the maturation and stimulus sensitivity of low threshold mechanoreceptors.

It has been known for some time that a small percentage of developing sensory neurons express the c-Kit receptor (Hirata et al., 1995; Hirata et al., 1993; Keshet et al., 1991). Here the inventors defined the neurochemistry of c-Kit+ neurons in the adult DRG. The inventors found that 20% of all DRG neurons expressed c-Kit, and most c-Kit+ neurons (>80%) were TrkA positive, neurofilament-heavy chain negative and also expressed SP and CGRP. These c-Kit+ neurons thus define a subpopulation of C-fiber nociceptors. In addition, the inventors show that about half of the c-Kit+ neurons also expressed the capsaicin gated ion channel TRPV1. In addition to these small diameter c-Kit+ sensory neurons, the inventors also identified a sub-population of larger sensory neurons that expressed c-Kit, which may be mechanoreceptive in nature. Earlier work showed that SCF promotes the survival of embryonic sensory neurons in culture, an effect that synergizes with the survival promoting effects of NGF (Hirata et al., 1993). However, the inventors show here that SCF/c-Kit receptor signaling in vivo is not required for the survival of the large majority of DRG neurons.

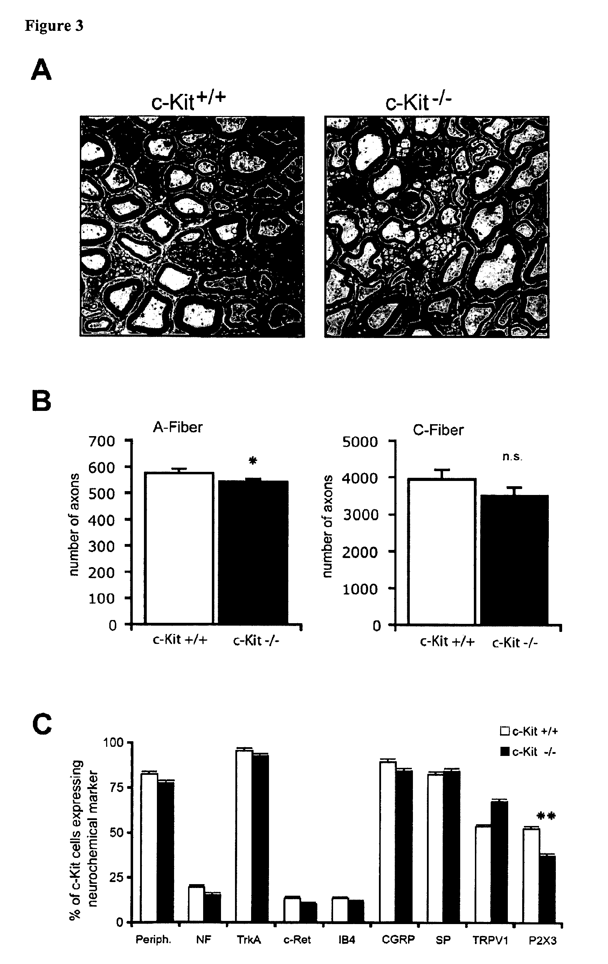

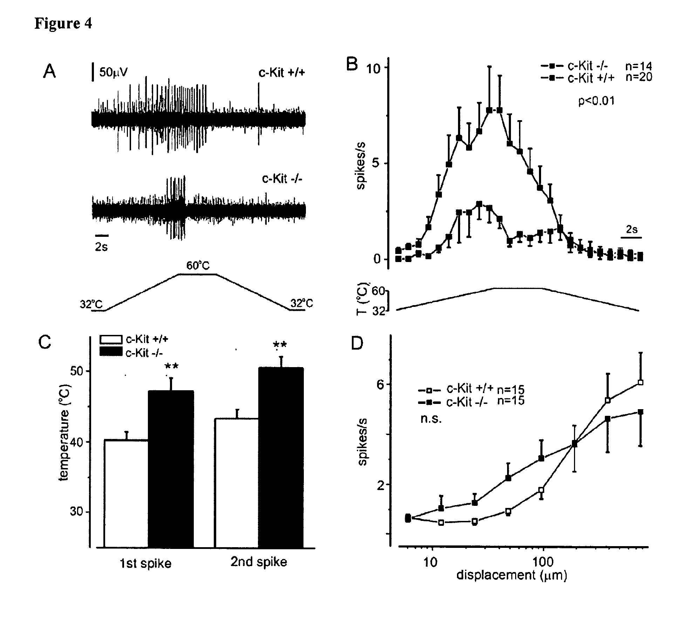

The inventors analyzed adult *c-Kit*^{*-*/*-*} mice to define the physiological role of this signaling system for sensory neurons. The inventors observed that *c-Kit*^{*-*/*-*} mice exhibit pronounced hyposensitivity to noxious heat applied to the hindpaw. This behavior could be ascribed to a pronounced reduction in the heat sensitivity of C-fiber polymodal nociceptors (Figure 4). Interestingly, the number of noxious heat sensitive C-fibers was not altered in *c-Kit*^{*-*/*-*} mice, but their average heat sensitivity, as assessed by firing rates to a heat stimulus, was reduced to less than a third of control C-fibers. Thus, signaling via the c-Kit receptor is essential to maintain normal noxious heat sensitivity of C-fibers, but the mechanosensitivity of these fibers remained normal. On closer inspection the inventors found that a sub-population (∼30%) of noxious heat sensitive C-fibers, which normally have very high rates of firing to heat (>60 spikes per heat stimulus), are especially susceptible to reductions in c-Kit signaling. Indeed even *c-Kit* haploinsufficiency is sufficient to reduce the noxious heat sensitivity of these fibers to that observed for the majority of "low responder" C_{MH} fibers (Supplementary Figure 3).

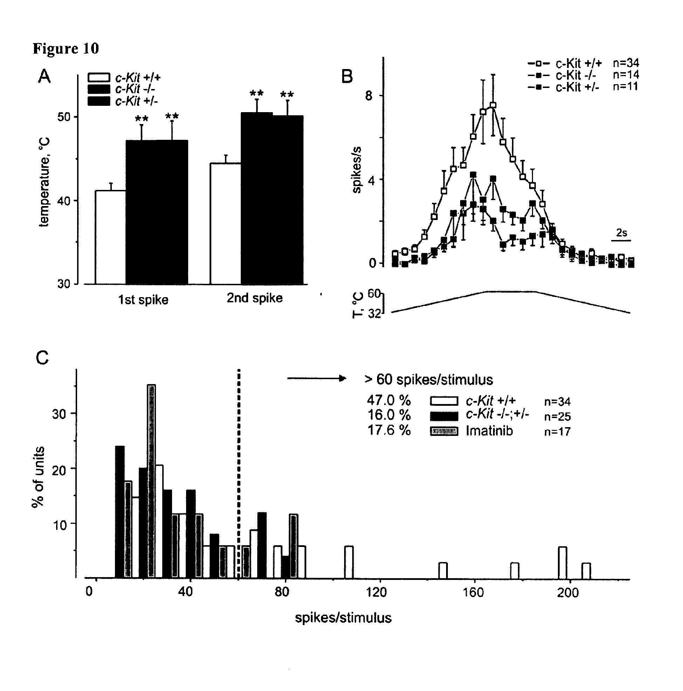

The inventors also treated adult mice with the c-Kit receptor inhibitor imatinib and found that this treatment essentially reproduced the effects of the *c-Kit* mutation on C_{MH} fibers (Figure 4; Figure 10). The observation that ∼30% of the C_{MH} fibers studied appear to be especially susceptible to alterations in c-Kit signaling suggests that expression of the c-Kit receptor in small diameter neurons is a marker of "high responding" C_{MH} fibers. These fibers may correspond to the 30% of TRPV1-positive neurons that in addition express c-Kit and respond to SCF.

As yet, c-Kit is the first example of a single gene being required for normal noxious heat sensitivity of C-fibers, and ablation of others, for instance the gene encoding TRPV1, does not appear to affect appreciably noxious heat sensitivity of C-fibers (Woodbury et al., 2004). However, immuno-depletion of NGF does result in a similar dramatic loss of noxious heat sensitivity in C-fibers (Bennett et al., 1998; Lewin and Mendell, 1994). A genetic analysis of the NGF/TrkA system has so far been precluded because it is essential for the survival of all nociceptive neurons and mutant mice are not viable after birth (Crowley et al., 1994; Smeyne et al., 1994). In contrast the present data reveal that c-Kit signaling regulates the functional properties of nociceptive neurons but is not required for their survival.

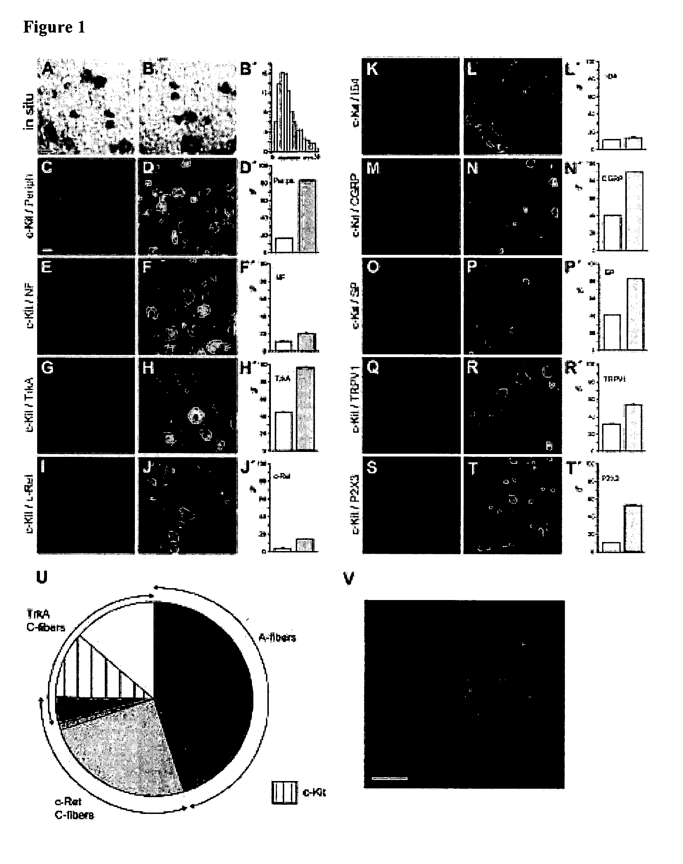

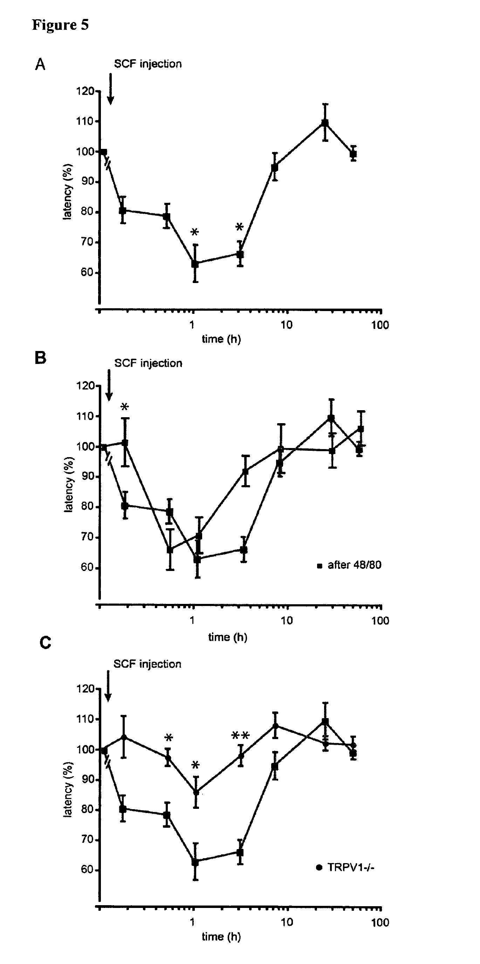

There are striking similarities between c-Kit and TrkA mediated regulation of noxious heat sensitivity. The presence of TRPV1 ion channels is required for the thermal hyperalgesia produced by activation of both c-Kit and TrkA receptors (Chuang et al., 2001) (Figure 5C). A sub-population of isolated sensory neurons in culture exhibit a specific noxious heat activated inward current called Iₕₑₐₜ (Cesare and McNaughton, 1996), and Iₕₑₐₜ is thought to be the basis of noxious heat sensitivity of polymodal nociceptors (C_{MH} receptors). Not only SCF, but also NGF, bradykinin, ATP and some cytokines are able to potentiate Iₕₑₐₜ in primary sensory neurons (Cesare and McNaughton, 1996; Galoyan et al., 2003; Kress and Guenther, 1999; Obreja et al., 2005). The effect on Iₕₑₐₜ is likely to account for the marked thermal hyperalgesia observed after acute administration of SCF to animals. The inventors also show that around 30% of TRPV1 positive neurons also possess c-Kit receptors as the capsaicin-induced [Ca2+]ᵢ increases can be potentiated in these cells by prior exposure to SCF. Indeed the proportion of capsaicin sensitive cells that are responsive to SCF matches very well the proportion of TRPV1 positive cells that possess c-Kit receptors (Figure 1). The effective concentration of SCF needed to produce significant potentiation was found to be in the picomolar range, as reported for NGF (Shu and Mendell, 1999) and similar to the effective concentrations of SCF that are found in other cellular assays (Tkaczyk et al., 2004).

The present data support a model in which up and down regulation of Iₕₑₐₜ by c-Kit activity is a central physiological mechanism for regulating noxious heat sensitivity. The signal transduction mechanisms through which activation of c-Kit regulates Iₕₑₐₜ and TRPV1 remain to be studied in detail. There is an extensive but controversial literature on the signaling pathways engaged by TrkA to sensitize Iₕₑₐₜ and TRPV1. Recent evidence however strongly suggests that TrkA activation of PI3kinase leads to TRPV1 phosphorylation and increased membrane insertion of the channel (Stein et al., 2006; Zhang et al., 2005).

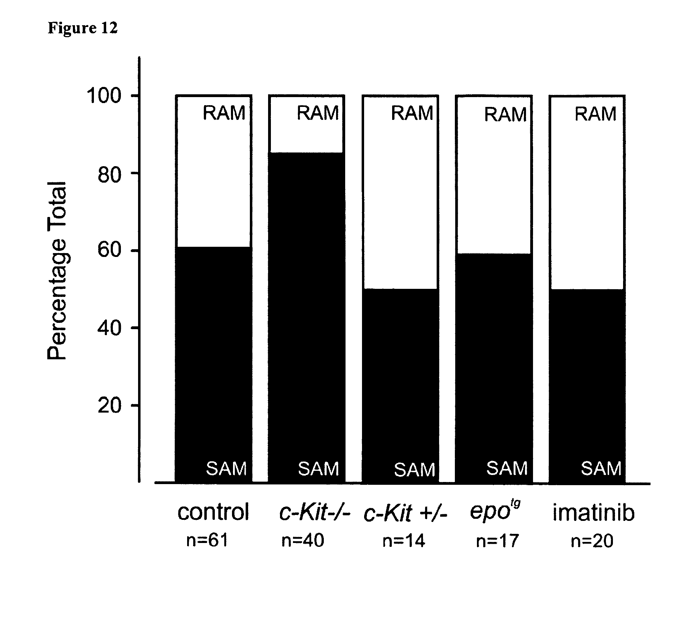

In addition to nociceptive neurons that respond to noxious heat, c-Kit is expressed in a second distinct population of putative mechanoreceptors (Figure 1). The second c-Kit+ population expresses heavy chain neurofilament, a marker of myelinated sensory neurons. We counted myelinated axons in the saphenous nerve of *c-Kit*^{*-*/*-*} mice, and found a small (5%) reduction in the number of axons compared to control nerves (Figure 3). An earlier study addressed the role of SCF and c-Kit in regulating sensory axon numbers using mice carrying hypomorphic c-Kit and SCF alleles (Lourenssen et al., 2000). These authors reported a substantial reduction in myelinated and unmyelinated axon numbers in thoracic cutaneous nerves. It is unclear if hypomorphic mutations in the SCF/c-Kit system lead to distinct physiological effects compared to null mutations. Alternatively, differences in the c-Kit-dependence of distinct peripheral nerves or differences in the genetic backgrounds of the mice used could account for the discrepancy. In the present application, electrophysiological studies revealed that the myelinated mechanoreceptor population was altered in *c-Kit*^{*-*/*-*} mice. First, fewer RAMs were found in the saphenous nerve and this may reflect a role of c-Kit in differentiation, and the development of more SAMs at the expense of RAMs in the mutant mice. The inventors also noted a striking change in the physiology of SAMs in *c-Kit*^{*-*/*-*} mice: mutant receptors discharged twice as many spikes to displacement stimuli than control receptors. In addition the inventors observed a lower mechanical threshold required to evoke spikes from both SAMs and AMs (Table 1). Thus, in the absence of SCF/c-Kit, the inventors observed a gain in function (increased mechanosensitivity) of sub-populations of sensory neurons. The number of mechanoreceptors affected is in agreement with the number of c-Kit+ large diameter sensory neurons, indicating that the alteration is a direct effect of the loss of c-Kit. For instance, c-Kit might down-regulate transduction components that set the normal mechanosensitivity of these neurons. As yet, only a few molecules required for sensory neuron mechanosensitivity have been characterized (Lewin and Moshourab, 2004; Wetzel et al., 2007). The inventors found no evidence that behavioral sensitivity to mechanical stimuli is altered after acute SCF injection (Figure 10). Consistent with the idea that the effects of c-*Kit* mutation on mechanoreceptors occur during development we did not observe any effect of imatinib on the incidence or functional properties of mechanoreceptors in the adult (Figure 12). The inventors therefore propose that SCF/c-Kit signaling is required during development to set the normal function of myelinated mechanoreceptors.

The small molecule c-Kit receptor inhibitor Gleevec® is a widely used anti-cancer drug that blocks the c-Kit receptor, and the inventors show here that Gleevec®, as a preferred example, can decrease sensitivity to pain in mice. Thus Gleevec may also have significant analgesic effects together with its potent anti-tumour activity. c-Kit is one of the oldest and best characterized receptor tyrosine kinases. Loss- and gain-of-function mutations in *c-Kit* have been reported to affect hematopoiesis and germ cell development. In addition, activating mutations or mis-expression of c-Kit are strongly linked to the occurrence of tumors like gastrointestinal stromal tumors. Recently, small molecules that effectively block normal c-Kit have been developed and shown to be clinically effective in the treatment of gastrointestinal stromal tumors (Wong and Witte, 2004). In the present invention the inventors have shown that one such drug (imatinib) can be used to down-regulate the noxious heat sensitivity of C-fibers in vivo (Figure 4). This data together with the characterization of the c-Kit function in nociceptors raises the exciting possibility that interfering with c-Kit signaling also produces effective analgesia in man.

The invention will now be further described in the following examples with reference to the accompanying drawings, without being limited thereto. For the purpose of the present invention, all references as cited herein as well as the sequence listing are incorporated by reference in their entireties. In the Figures,

**Figure 1** shows the in situ and immunohistochemical analysis of c-Kit expression in lumbar DRGs of adult mice. A, B, non-radioactive in situ hybridization, showing expression of c-Kit in small and medium sized sensory neurons (two adjacent fields). B', size distribution, showing the percentage of c-Kit+ cells with a given diameter (binning analysis, 2 µm increments). C-T, immunohistochemical analysis of c-Kit protein expression (red, left and right panels) in neurons co-stained with the following neurochemical markers (green, right panel only): C, D, peripherin, E, F, heavy chain neurofilament; G,H, TrkA; I,J, c-Ret; K,L, isolectin B4; M,N, CGRP ; O,P, SP; Q,R, TRPV1; S,T, P2X3. D'-T', quantification of immunopositive populations, showing percentage of neurons positive for the given neurochemical marker that also co-express c-Kit (white bar), or the percentage of c-Kit+ neurons that co-express the given neurochemical marker (grey bar). Bars, A-T, 20 µm. U, pie chart showing the distribution of c-Kit+ neurons in relation to the entire population in the DRG. Fibers expressing c-Kit (red hatch) are depicted as proportions of total C-fibers (white, TrkA+; light grey, c-Ret+; medium grey, c-Ret+/TrkA+) or myelinated afferents (dark grey, A-fibers). V, SCF immunoreactivity in the suprabasal keratinocyte layer of the epidermis, SCF is green and red is a nuclear stain. Scale bar is 50µm.

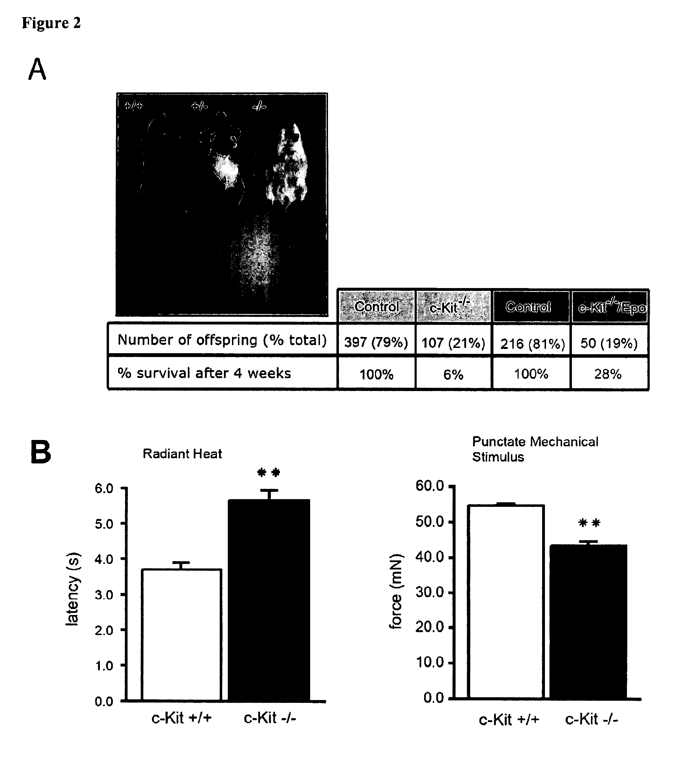

**Figure 2** shows the behavioral analysis of adult mice lacking a functional c-Kit signaling system. A, external appearance of wildtype, heterozygous and homozygous adult c-Kit mutants, and percentage of control (+/+, +/-) and mutant offspring (-/-) resulting from intercrosses of the *c-Kit*+/- mutant substrain (*WB*) or after introducing an erythropoietin-expressing transgene (*c-Kit*^{*-*/*-*};*Epo^{tg}*, dark grey column header). B, behavioral testing of adult control (white bars) and *c-Kit*^{*-*/*-*} mutant (blue bars) mice. Data show latency for paw withdrawal to radiant heat (left) or the mechanical threshold required for withdrawal from a vertically-applied punctate mechanical stimulus (right). **indicates p<0.0001 unpaired t-test.

**Figure 3** shows the sensory neuron number and neurochemistry in *c-Kit* mutant mice. A, electron micrographs of sections of saphenous nerve in control and mutant mice. B, quantitation of myelinated axons (A-fibers) and non-myelinated axons (C-fibers) in the saphenous nerves of control (white bars) or *c-Kit*^{*-*/*-*} mice (blue bars) (*, P<0.05). C, quantification of the percentage of c-Kit+ DRG neurons co-expressing different neurochemical markers in control (white bars) or *c-Kit*^{*-*/*-*} mutant mice (blue bars). The numbers of neurons in mutant mice expressing P2X3 were decreased by 28% (p<0.001, unpaired t-test).

**Figure 4** shows the electrophysiological analysis of sensory neurons in adult control and c-*Kit^{-l-}* mice. A, response of mechano-heat sensitive C fiber afferents (C_{MH}) to a heat stimulus applied to the skin (temperature ramp from 32°C to 60°C and return to 32°C over time, as indicated), upper panel, single unit recording from a control mouse, lower panel, single unit from a *c-Kit*^{*-*/*-*} mouse. B, quantification of the number of spikes per 1 second time window during application of the heat ramp in controls (black squares); in *c-Kit* mutants (dark blue squares), or imatinib treated adult mice (light blue squares) the curves were significantly different from control (Two way ANOVA F=7.67 p<0.01). C, the average temperature threshold required to elicit the first or second spike in C_{MH} fibers in control (white bar), *c-Kit* mutants (dark blue bars), or imatinib treated mice (light blue squares), **, P<0.001 unpaired t-test). D, spiking rates of C_{MH} fibers recorded in control (open squares) and *c-Kit* mutant mice (dark blue squares) to a series of increasing mechanical displacements of the receptive field (not significantly different, two way ANOVA test).

**Figure 5** shows the behavioral analysis of the effect of c-Kit activation (SCF, i.p., arrow) on paw withdrawal latencies (% of baseline) in response to radiant heat. A, Injection of SCF into C57B1/6 wildtype mice (red squares, see also B, C). B, injection of SCF into mice pretreated for three days with the mast cell degranulating agent 48/80 (black squares). C, injection of SCF into homozygous mutant mice lacking the cation channel TRPV1 (black squares). Time points where significant differences are observed between controls and *TRPV1*^{*-*/*-*} mice or after mast cell degranulation are indicated, * p<0.05; ** p<0.001.

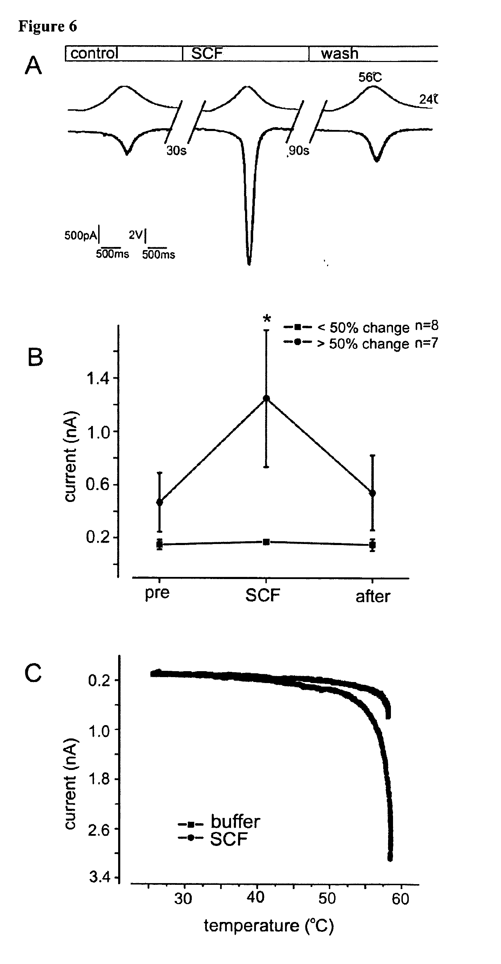

**Figure 6** shows the potentiation of Iₕₑₐₜ and TRPV1 in DRG neurons by SCF. Electrophysiological analysis of Iₕₑₐₜ in cultivated small diameter sensory neurons derived from adult wild type mice. A, inward currents (black trace) in response to a standard heat stimulus (red trace), before, during addition of SCF, and after washout, as indicated. B, quantification of heat-induced inward currents distinguishes two neuronal populations, one that shows robust potentiation of inward currents in response to SCF (10 nM) (red circles), (*p<0.01 Wilcoxin test on ranks), the other set of cells remains unresponsive to SCF (black squares). C, representative trace of inward current magnitude plotted against temperature for one cell, prior to (black trace) and during SCF treatment (red trace).

Results from calcium imaging experiments (not shown Fig 6 D-F): Example traces of [Ca2+]ᵢ transients in a single cell in response to two capsaicin puffs, top a control cell with intervening pulse of buffer solution, note the marked decrease in [Ca2+]ᵢ increase to the second capsaicin puff. Bottom, increased [Ca2+]ᵢ to the second capsacin puff after prior exposure of the cell to 10nM SCF. E, The proportion of cells with a significant potentiation of [Ca2+]ᵢ. Control indicates no intervening SCF stimulus together with a series of increasing SCF concentrations ranging from 0.001 to 100 nM. The response to the second capsaicin was puff was also measured immediately following the SCF stimulus (10nM, short). All SCF concentrations of 0.1 nM and above produced a significant increase in the proportion of sensitized cells compared to control (Fishers exact test p<0.05). F, same data are shown in E but the data from all cells tested are shown as percentage change compared to the first capsaicin induced [Ca2+]ᵢ transient. Note the wide range of responses among SCF responsive cells.

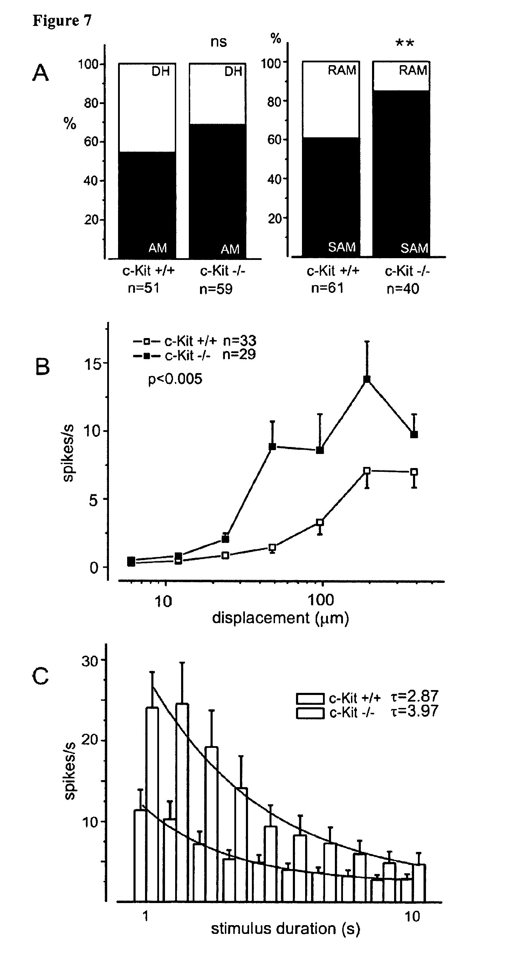

**Figure 7** shows the mechanoreceptors in *c-Kit* mutant mice. A, proportions of myelinated D-hair and thinly-myelinated mechanoceptors (left panel) and large diameter myelinated mechanoreceptors (RAM, rapidly adapting; SAM, slowly-adapting; right panel). Reduction in the proportion of RAMs from 40% of all Aβ fibers to around 15% of the total in *c-Kit* mutant mice **P<0.01 Chi-squared test). B, spiking rates of SAMs in control (black squares) and c-*Kit* mutants (blue squares) in response to a series of ascending mechanical displacements of the skin, two way ANOVA F=10.2, p<0.005. C, adaptation of SAMs during the static phase of the displacement stimulus (192µm). The time dependent reduction in firing rate was fitted with a single exponential function and time constant τ calculated for controls and mutants afferents. The adaptation time constant was comparable in mutant and control mice.

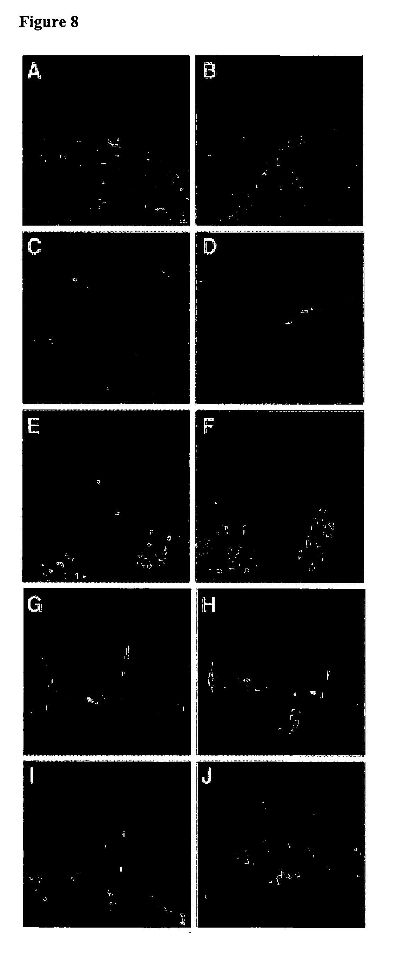

**Figure 8** shows the immunofluorescence analysis of nerve innervation in the skin from c-Kit controls (A, C, E, G, I) and c-Kit-/- mice (B, D, F, H, J). Neurochemical markers (green) were detected using antibodies against SP (A, B), calbindin (C, D), peripherin (E, F), PGP9.5 (G, H), or CGRP (I, J). Nuclei (red) were detected with TOTO3 stain (Molecular Probes). Panels A-F are low magnification views showing the dermis and epidermis, panels G-J are higher magnifications at the dermal/epidermal border to show cutaneous free nerve endings in more detail.

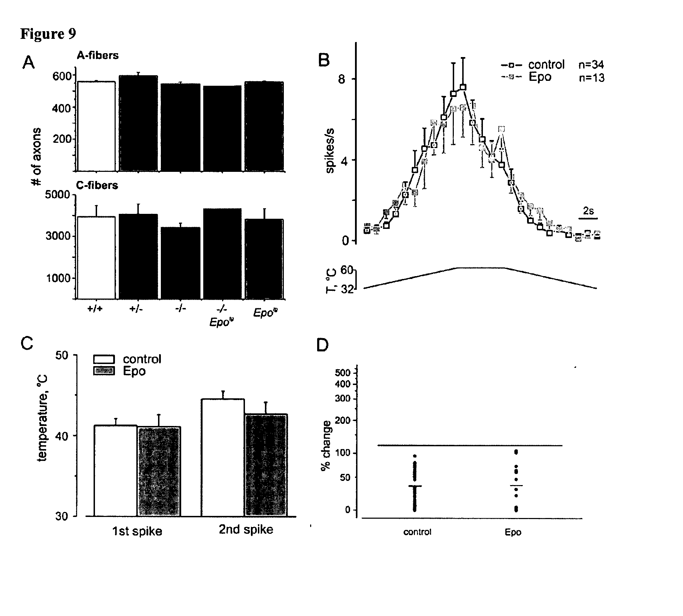

**Figure 9** shows that the *Epo^{tg}* has no effect on the number or functional properties of C-fiber nociceptors. A, The mean numbers of myelinated (A-fiber, top) and unmyelinated (C-fiber, bottom) axons counted in the saphenous nerve from mice with *c-Kit* mutation with or without the *Epo^{tg}.* Note that data was obtained from just one mouse with *c-kit-*/*-; Epo^{tg}* and so no error bars are shown for this bar (errors are s.e.m.). No significant differences were found between any of the genotypes tested both for A and C-fiber axon number. B, Mean firing rates of C-fiber polymodal nociceptors in response to a noxious heat stimulus recorded in control wild type (open squares) and *c-kit* +l+*;Epo^{tg} (*closed grey squares). No significant difference in the firing behavior of C-fibers was found between these two genotypes (two-way ANOVA analysis). C, Mean temperature threshold for first and second spike is plotted for control wild type (no fill) and *c-kit* +/+*;Epo^{tg} (*grey filled bars). There was no significant difference between polymodal nociceptors threshold between the two genotypes. D, Percentage change in the magnitude of the capsaicin evoked [Ca2+]ᵢ increase following a second capsaicin puff. Intervening stimulation with recombinant erythropoietin (10µM) had no significant effect on the capsaicin evoked desensitization of the response.

**Figure 10** shows that *c-kit* haploinsufficiency is sufficient to down regulate the noxious heat sensitivity of C-fiber nociceptors. A, Mean temperature threshold for the first and second spike in nociceptors recorded in *c-Kit* +/- mice (light blue bars) was elevated to a similar degree to that found in *c-Kit-*/*-* mice (dark blue bars), **p<0.001, unpaired t-test. B, Mean firing rates during the ramp for nociceptors recorded in *c-Kit* -/- (filled dark blue squares) and *c-Kit* +/- mice (filled light blue squares). Note that the firing rates of C-fibers in both genotypes are significantly reduced compared to control (p<0.05 Two-way ANOVA). C, Evidence that a sub-population of "high responding" C-fibers are the susceptible to reduction c-kit signaling. The distribution of mean response (total spikes per stimulus) magnitude was plotted for all C-fibers studied. Note that among control C-fibers 47% of the fibers responded with more than 60 spikes/stimulus (open bars). In the mice treated with imatinib (light blue bars) or mice with a *c-kit* gene mutation (*c-Kit-l-;c-Kit* +/-, dark blue bars) many fewer fibers (∼16%) had responses >60 spikes/stimulus. The difference between the percentage of high responders in the control and *c-Kit* mutant mice (47%-16=31% approximates to the number of SCF responding cells found in calcium imaging experiments.

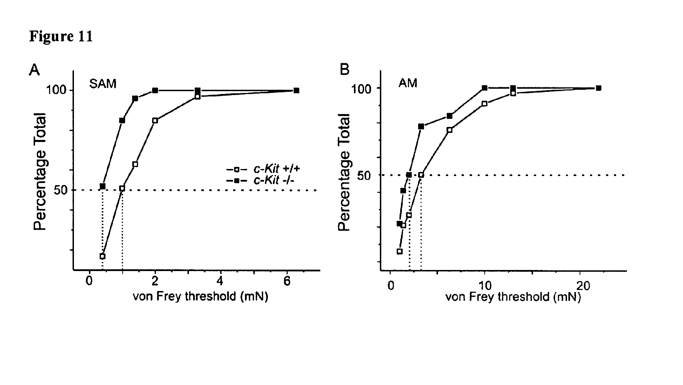

**Figure 11** shows that von Frey hair thresholds of mechanoreceptors are altered in c-Kit mutant mice. Cumulative sum plots of the von Frey threshold for SAM (A) and AM receptors (B). The dotted line indicates the median threshold for the receptor population measured. Mann-Whitney test indicated that the median threshold were significantly different (p<0.01).

**Figure 12** shows that only null mutation of the c-Kit receptor alters the make-up of A-fiber mechanoreceptors. The proportion of RAM and SAM receptors recorded is plotted for *c-Kit* +/+, -/-, +/-, +/+; *Epo^{tg}* and imatinib mice. The only significant change in the proportion or RAM and SAM receptors was seen in c-kit-/- mice (p<0.05; Chi-squared test).

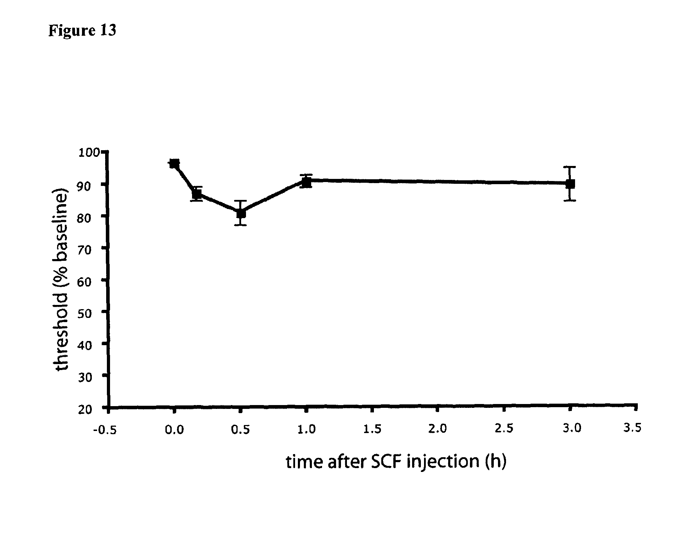

**Figure 13** shows injection of SCF at doses that produce thermal hyperalgesia did not alter behavior to punctate mechanical stimulation.

### Examples

### Materials and methods

*C-Kit*^{*W*/+} mice on the inbred WB-strain, isogenic with C57B1/6, were originally established at the Jackson Laboratory and obtained via Hans-Reimer Rodewald (Department of Immunology, Ulm, Germany) from Shizuoka Laboratory Company (SLC, Shizuoka, Japan). *C-Kit*^{*W*/*W*} mutants generated through inbreeding of the *c-Kit*^{*W*/+} colony were obtained at a low frequency. In addition, transgenic male mice overexpressing erythropoietin (*Epo^{tg}*,) with a 12-fold increase in plasma erythropoietin levels (Ruschitzka et al., 2000) were crossed with c-*Kit*^{*W*/+} mice to generate compound hetero/hemizygous male mice as described previously (Waskow and Rodewald, 2002). Softened food and nutrient agar were supplied to litters containing homozygous *c-Kit*^{*W*/*W*} offspring to aid survival. The *c-Kit^{W}* allele is characterized by a splice-site mutation which results in exon skipping and production of a functionally inactive c-Kit protein that lacks the transmembrane domain. The presence of the *c-Kit^{W}* mutation in our colony was confirmed by *Hph*I digestion of a PCR fragment spanning the splice site mutation (Hayashi et al., 1991). The TRPV1^{-/-} strain were obtained from the Jackson laboratory and like the c-Kit^{W/W} mice was on a C57B1/6 background. The inventors treated adult C57B1/6 mice with a daily sub-cutaneous dose of imatinib mesylate (0.1mg/g; Sequoia Research Products Ltd. U.K.) over a period of 6-7 days.

### In situ hybridization, histology and immunohistochemistry

DRGs were freshly frozen in OCT compound (Sakura). Slides of 10-12 µm were postfixed for 10 min. and standard immunohistochemistry or *in situ* hybridization protocols using DIG-labelled probes were employed (Britsch et al., 1998). The inventors used the following antibodies: rabbit anti-TrkA (1:2000; gift from L. Reichardt, San Francisco, USA), goat anti-c-Ret (1:2000; R&D Systems, MN, USA), mouse anti-neurofilament (1:1000; Sigma, ST. Louis, MO, USA), rabbit anti-calcitonin gene-related peptide (1:20000; Sigma, ST. Louis, MO, USA), rabbit anti-TRPV1 (1:200; Santa Cruz, Santa Cruz, CA, USA), rabbit anti-P2X3 (1:5000; Abcam, Cambridge, MA, USA), rabbit anti-Peripherin (1:5000; Chemicon, Temecula; CA, USA), rabbit anti-Substance P (1:1000; Zymed, San Francisco, CA, USA), monoclonal rat anti-c-Kit (clone ACK2, 1:1000, Bioscience, San Diego, CA, USA), goat anti-SCF (R&D Systems, 2µg/ml), FITC-conjugated *Griffonia simplicifolia* IB4 lectin (1:200; Sigma, ST. Louis, MO, USA); secondary antibodies conjugated with Cy2, Cy3 or Cy5 were purchased from Jackson ImmunoResearch, West Grove, PA, USA). In the case of SCF staining in the skin control experiments in which the anti-SCF antibody were pre-incubated with 20µg/ml recombinant SCF showed no significant staining in the epidermis. Immunofluorescent preparations were examined with a Zeiss LSM 5 Pascal confocal microscope and images were processed in Adobe Photoshop.

### Electron microscopy

Electron microscopy Nerves were isolated from animals perfused with 4% paraformaldehyde and 2.5% glutaraldehyde in phosphate buffer, postfixed and contrasted with osmium tetroxide as described (Garratt et al., 2000). For light microscopy, nerves were embedded in Technovit 7100 resin (Heraeus Kulzer, Wehrheim, Germany); semi-thin sections (1µm) were stained with toluidine blue, and used to determine the numbers of myelinated axons. The numbers of non-myelinated axons in the saphenous nerve of wild type and mutant animals were determined by counting of twelve random fields under the electron microscope, and normalizing to total cross-sectional nerve area observed in semi-thin sections.

### Behavioral experiments

Paw withdrawal test. In the radiant paw-withdrawal test the mice were placed in a Plexiglas chamber (15 cm diameter, 22.5 cm in height). The stimulus was a high intensity beam directed at the plantar surface of the hindpaw; movement of the paw away from the beam results in the heat source being terminated, with the withdrawal latency indicated on a digital screen (Ugo Basile, Milan, Italy). Paw withdrawal latencies were determined alternately from both hindpaws (stimuli given every 30- 40 s). Criteria for inclusion of a data point were calm behavior of the mouse just prior to testing, and a clear paw withdrawal from the hot beam associated with paw flicking and grooming. Baseline values were taken once a day at the same time for a period of 3 days. c-Kit ligand (Stem Cell Factor, SCF, R&D Systems) was injected once intraperitoneally (1 mg/kg i.p.) into *TRPV1 -l-* and control C57B1/6 mice (adult females, 6- 8 weeks, 20-25 g body weight), and response latencies to radiant heating of the hind paw was measured at various times after injection as described.

Punctate mechanical stimulus. The sensitivity to punctate mechanical stimuli was assessed using the dynamic plantar aesthesiometer (Ugo Basile, Milan, Italy). Each mouse was placed in a Plexiglas chamber (15 x 15 x 22.5 cm, wire mesh floor). 10 minutes later a mechanical stimulus (a small diameter blunt metallic filament) was applied to the plantar surface with an increasing vertical force (continuous increase from 0- 70 mN in 2 s) and the withdrawal latencies were measured.

Statistical Analysis. All results are presented as mean ± SEM of at least 10 animals and analyzed by a Student's *t*- test in unpaired series to compare scores between WT and c-Kit ^{-/-}mutant animals. In all cases the significance level was P< 0.05.

### Cell culture

DRG neurons were isolated from adult mouse and cultured as previously described (Hu and Lewin, 2006). No NGF or other neurotrophin was added to the medium.

### Whole cell patch clamp recordings from isolated DRG neurons

Whole cell recordings were made from DRG neurons 24-48 h after plating using fire polished glass electrodes with a resistance of 4-7 MΩ. The recording chamber was perfused with extracellular solution containing (in mM) 140 NaCl, 1 MgCl₂, 2 CaCl₂, 4 KCl, 4 glucose, 10 HEPES, pH 7.4 and electrodes were filled with solution containing (in mM) 110 KCl, 10 Na⁺, 1 MgCl₂, 1 EGTA, 10 HEPES, pH7.3. Membrane current and voltage were amplified and acquired using an EPC-9 amplifier sampled at 10-40 kHz. For the whole cell recording, the membrane voltage was held at -60mV. Heat ramp stimuli (24-56°C) and drugs were applied using an automated perfusion system (WAS02, (Dittert et al., 2006). The outlet of the output capillary (400 µm inner diameter) with heating Cu-coil was placed 100 µm away from the patched cells. The SCF (10 nM) was dissolved in extracellular solution. Acquired traces were analysed using Pulse and PulseFit software (HEKA).

### Calcium Imaging Experiments

The inventors used standard Fura-2 based ratiometric calcium imaging techniques to record responses to application of capsaicin solutions in cultivated DRG neurons from the mouse. An inverted microscope (Zeiss Axiovert200) equipped with MetaView photonics imaging system, including the polychrome V, a CCD camera and the imaging software MetaView was used for cell imaging. Paired images (340 and 380 nm excitation, 510 nm emission) were collected every 1.7 seconds. Cells were cultivated overnight before calcium imaging experiments. For each recording a fresh coverslip was used. The SCF, capsaicin (1µM), and erythropoietin (1µg/ml) were diluted in extracellular buffer. Just those cells with a robust response to 40mM KCl were analysed. SCF or Erythropoietin was applied for 34 seconds and 272 seconds prior to the second capsaicin pulse (5 seconds).

### Skin nerve preparation

An in vitro skin nerve preparation (Koltzenburg et al., 1997; Wetzel et al., 2007) was used to record from functionally single primary afferents. Mice were sacrificed by placing them in a chamber gassed with CO₂ and the saphenous nerve was dissected together with the innervated skin attached. The skin was placed corium side up in the oxygenated SIF bath (123 NaCl, 3,5 KCl, 0.7 MgSO₄, 1.7 NaH₂PO₄. 2.0 CaCl₂, 9.5 sodium gluconate, 5.5 glucose, 7.5 sucrose and 10 HEPES, in mM) at 32°C, and the nerve was desheathed and teased into microfilaments to enable single unit recording. Units were classified according to their conduction velocities, von Frey thresholds and firing properties. *c-Kit* mutant mice were significantly smaller than wild type littermates and conduction velocities measured for myelinated fibers were slower than controls but comparable to those found in younger wild type mice (3-4 weeks) (Supplementary Table 1). The conduction velocity cut-off between Aβ and Aδ fibers was determined to be 9 m/s in c-Kit mutants based on measurements of the Aβ and Aδ wave in compound action potential recordings as previously described (Koltzenburg et al., 1997). Receptive fields were found using mechanical stimulation with a glass rod and units were tested both with ascending series of displacement stimuli ranging from 6 to 768 µm and with ascending series of ramp velocities, from 0.012 to 2.9 m/s, with constant displacement. A computer controlled linear stepping motor (Nanomotor Kleindiek Nanotechnik) was used to apply standardized mechanical stimuli. C-fibers were tested for a heat response using a contact thermal stimulator (Yale University, Instrumentation Repair and Design Shop). Heat ramp stimuli (32-60°C) were applied at the rate 2°C/s, keeping the temperature at 60°C for five seconds and cooling it down to the bath temperature at the same rate. The raw electrophysiological data were collected with Powerlab 4.0 system and analyzed off line with the spike histogram extension of the software.

### Results

### c-Kit receptors in nociceptive sensory neurons

The inventors first characterized the distribution and neurochemistry of c-Kit positive neurons within the DRG using in situ hybridization and immunohistochemistry (Figure 1). The inventors could detect c-Kit mRNA in about 20% of DRG neurons, primarily small diameter putative nociceptors, as well as in a subpopulation of middle to large sized DRG neurons (Figure 1A, B, B'). The inventors employed double immunofluorescence using a monoclonal antibody directed against c-Kit together with antibodies against other markers to define further the neurochemical phenotype of c-Kit positive neurons. The inventors found that around 80% of c-Kit+ neurons were of small size and positive for peripherin, which is a good marker of unmyelinated C-fibers (Goldstein et al., 1991), (Figure 1C, D, D'). The remaining 20% of c-Kit+ cells were somewhat larger and were positive for heavy chain neurofilament which is characteristic of cells that are myelinated (Figure 1E, F, F'). Thus, it appears that two populations of c-Kit+ cells exist, the majority being nociceptors and a smaller proportion being myelinated mechanoreceptors.

Small diameter nociceptors can be divided into two major neurochemical classes, those that express the NGF receptor TrkA and the pain related neuropeptides calcitonin gene-related peptide (CGRP) and substance P (SP), and neurons that largely lack neuropeptides, but express the c-Ret receptor tyrosine kinase. The latter sub-population of nociceptors can also be defined by their cell surface binding of the isolectin B4 (IB4) and expression of the ionotropic P2X3 receptor (Snider and McMahon, 1998). The inventors found that among small diameter sensory neurons c-Kit protein was largely restricted to the TrkA population. Thus, the majority of c-Kit+ sensory neurons (>95%) expressed TrkA (Figure 1G, H, H'), while small numbers were positive for c-Ret (Figure 11, J, J') or exhibited IB4 binding (Figure 1K, L, L'). Consistent with this, most c-Kit+ neurons were peptidergic, as determined by co-expression of CGRP (Figure 1M, N, N'), or SP (Figure 1O, P, P'). The inventors also found that more than half of the c-Kit+ cells expressed the cation channel TRPV1 or the purine receptor P2X3 (Figure 1Q, R, R', S, T, T'). Since many c-Kit+ neurons are P2X3 positive they may be identical with an appreciable population of TrkA positive neurons that also express P2X3 (Ramer et al., 2001). In summary, c-Kit defines a sub-population of peptidergic TrkA+ neurons, predominantly small diameter nociceptors, the majority of which express the TRPV1 and P2X3 ion channels.

Since it was clear that a sub-population of C-fiber nociceptors express the c-Kit receptor the inventors also asked if its ligand SCF is normally present in the skin of adult mice. The inventors found specific immunoreactivity for SCF in the upper layers of the epidermis (Figure 1V), an area of the skin that is richly innervated by peptidergic C-fibers (Kruger et al., 1989).

### Heat nociception is impaired in c-Kit mutant mice

The expression pattern of c-Kit prompted the inventors us to undertake a genetic analysis of its function in somatic sensation. The inventors employed a mouse strain harboring the original dominant-spotting *W* allele, first recognized in the early 1900s by Durham (Durham, 1911) and defined genetically by Little as having a lethal effect when homozygous (Little, 1915; Rifaat, 1954). This allele results from a point mutation in the 5' intron splice donor site downstream of the exon encoding the transmembrane domain (Hayashi et al., 1991). Previous analyses of dominant-spotting mice have demonstrated that the *W* allele is a null (Nocka et al., 1990). The inventors used a specific inbred substrain of the *c-Kit*^{*W*/+} mouse line (WB-pedigree, Jackson Labs) established during an inbreeding procedure in which parental animals were selected on the basis of enhanced postnatal survival in their homozygous mutant c-*Kit*^{*W*/*W*} offspring (*W*A, *WB*, *W*C and *W*D pedigrees, see also (Russell and Lawson, 1959). Crosses between heterozygous *c-Kit*^{*W*/+} mice from the WB strain of animals yielded a small proportion (6%) of viable *c-Kit*^{*W*/*W*} mutant mice surviving for longer than four weeks (Figure 2A). To increase further the number of adult *c-Kit*^{*W*/*W*} mutants, the inventors used a transgenic rescue strategy to overcome the lethal anemia in *c-Kit*^{*W*/*W*} mutants. The inventors crossed a transgenic mouse line overexpressing erythropoietin into the *c-Kit*^{*W*/+} strain (Ruschitzka et al., 2000) and the resulting progeny were then interbred, resulting in an increase in the proportion of surviving *c-Kit* mutants to 28%. The inventors refer to mutant *c-Kit*^{*W*/*W*} and *c-Kit*^{*W*/*W*}; *Epo^{tg}* animals as *c-Kit^{-l-}*in the rest of the present application.

The inventors first analyzed *c-Kit^{-l-}* mice in behavioral tests of acute thermal and mechanical nociception. Mice lacking c-Kit function demonstrated a marked hypoalgesia to radiant heat stimuli, with an average increase of 40% for foot withdrawal latency compared to wild type littermates (p<0.001 unpaired t-test) (Figure 2B). In contrast, in a test of mechanical nociception, the force threshold for foot withdrawal of *c-Kit*^{*-*/*-*} mutants was decreased by on average 20% compared to control mice (p<0.001 unpaired t-test) (Figure 2B). Such altered responses to acute nociceptive stimuli may have several underlying causes, including neuronal loss, or altered innervation patterns of the skin or dorsal spinal cord. The inventors analyzed semi-thin sections and ultrathin electron micrographs of the saphenous nerve, a pure cutaneous nerve innervating the skin of the lower hindlimb, to determine the numbers of non-myelinated (C-fibers) and myelinated (Aδ nociceptors and Aβ mechanoreceptors) axons. No major changes in the number of fibers in *c-Kit*^{*-*/*-*} mutant animals were observed (Figure 3A, B). In addition, immunohistological analyses of skin preparations revealed a normal innervation of the dermis, epidermis and other target tissues (e.g. arterioles, muscle) in mutant mice (see Figure 8). The *c-Kit^{W}* allele permits the detection of a mutant c-Kit protein, although this is expressed at lower levels than the normal gene product (Nocka et al., 1990; Waskow et al., 2002). The neurochemical nature of c-Kit+ neurons in mutant mice was unchanged, except the proportion of c-Kit+ neurons expressing the purine-activated channel P2X3 was reduced in mutant mice (28% reduction, p<0.001, unpaired t-test) (Figure 3C). It has been shown that erythropoietin may itself have neuroprotective effects when administered to mice (Campana and Myers, 2003). However, the inventors' anatomical analyses were carried out on both *c-Kit*^{*-*/*-*} and *c-Kit*^{*-*/}*⁻ ;Epo^{tg}* mice and the counts of A-fiber and C-fiber axons were not different between animals with or without the *Epo^{tg}* (Figure 9A). These results suggest that erythropoietin overexpression has little influence on normal programmed cell death in the mouse.

The lack of neuronal cell death and the normal innervation patterns of the skin and spinal cord suggested that the marked changes in acute pain behavior in *c-Kit* mutants were due to functional alterations in primary afferent nociceptors. The inventors analyzed the physiological properties of individual sensory neurons of *c-Kit*^{*-*/*-*} mutants using the isolated skin-nerve preparation. Due to the marked thermal hypoalgesia of *c-Kit*^{*-*/*-*} mutant mice, the inventors first focused on examining the response properties of noxious heat-sensitive polymodal nociceptors. The inventors recorded from a total of 72 C-fiber nociceptors in control and *c-Kit*^{*-*/*-*} mice and noted that the proportion of noxious heat sensitive neurons (so called C-mechanoheat, C_{MH} fibers) found in the mutant was not significantly altered compared to control (68% in *c-Kit*^{*-*/*-*} compared to 72% in wild type, see Table 1). However, the C_{MH} fibers recorded in *c-Kit*^{*-*/*-*} mutants displayed a substantial reduction in their sensitivity to noxious heat (Figure 4A). This was reflected in a large reduction in the average spike rate of C_{MH} fibers in *c-Kit*^{*-*/*-*} mice in response to a standard heating stimulus of the receptive field (Figure 4B). The average number of spikes from C_{MH} fibers in *c-Kit* mutants was 4.5-fold smaller than that found in wild type neurons (mean spike count to the heat stimulus 14.1 ± 2.5 spikes in *c-Kit^{-l-}* mutants compared to 65.9 ± 9.5 spikes in controls, p<0.001 unpaired t-test). Not only was the total response reduced, but the temperature threshold for the first and second spike was elevated by between 5° and 7° C in *c-Kit*^{*-*/*-*} mice compared to controls (Figure 4C). The inventors then went on to examine the properties of C-fiber nociceptors in *c-Kit*^{+/*-*} mice to test if the functional deficit in C-fibers is observed with *c-Kit* haploinsufficiency. Interestingly, the inventors also observed a reduction in noxious heat sensitivity of C_{MH} fibers in *c-Kit*^{+/*-*} mice that was quantitatively indistinguishable from that observed in *c-Kit*^{*-*/*-*} mice (Figure 10A, B). In cutaneous nerves C_{MH} fibers make up 60-70% of all nociceptors and so it was initially puzzling how deletion of the c-Kit receptor, which is expressed in around 20% of C-fibers, could lead to such a large reduction in the noxious heat sensitivity of C_{MH} fibers. However, the inventors found that when the distribution of the total heat response of individual units in control mice was plotted, around 47% of the units could be characterized as "high responders" (>60 spikes/stimulus) (Figure 10C). An analysis of the data from mice with *c-Kit* mutations revealed that the majority of these "high responder" C_{MH} fibers were essentially absent. This data suggests that a subpopulation of "high responding" C_{MH} fibers require c-Kit signaling to maintain their normally high sensitivity to noxious heat (Figure 10C).

The reduction in C_{MH} fiber noxious heat sensitivity that the inventors observed in *c-Kit*^{*-*/*-*} mice might conceivably arise as a result of the overexpression of erythropoietin in some of the mice examined. In order to control for this possibility the inventors made an extensive analysis of single C-fiber properties in *c-Kit*^{+/+} ;*Epo^{tg}* mice which had been derived from matings between *Epo^{tg}* and *c-Kit*^{+/*-*} mice. However, the inventors found that the physiological properties of C_{MH} fibers including spike rates and heat thresholds were essentially identical to those found in *c-Kit* wild type mice (Figure 9B, C).

The spiking rates of C-fiber nociceptors and myelinated AM-fibers to a series of supra-threshold mechanical stimuli were not significantly altered (Figure 4D). However, the median von Frey hair force (vFT) required to evoke spikes from myelinated AM fibers in *c-Kit*^{*-*/*-*} mice was significantly lower than that found in controls (median vFT, 2.5 mN in *c-Kit*^{*-*/*-*} mice compared to 3.6 mN in control, p<0.01, Mann-Whitney U-test see also Figure 11 and Table 1). The effect on AM mechanosensitivity might account for the observation that paw withdrawal threshold was lower in *c-Kit*^{*-*/*-*} mice (Figure 2B).

Thus far, our data did not show that c-Kit signaling in the adult animal is necessary for maintaining noxious heat sensitivity. For example, it is also feasible that SCF/c-Kit signaling during development sets the heat sensitivity of polymodal nociceptors. To address this issue, the inventors used imatinib (also called STI571 and Gleevec®, Novartis Pharmaceuticals) which is a potent c-Kit receptor blocker. The inventors treated mice with clinically effective doses of imatinib (0.1 mg/g administered sub-cutaneously) for 6-7 days (Kerkela et al., 2006) and then recorded the noxious heat sensitivity of C-fiber nociceptors. This treatment produced a reduction in C-fiber heat sensitivity that was almost identical to that found in *c-Kit*^{*-*/*-*} and *c-Kit*^{+/*-*} mice (Figure 4B,C; Figure 10C). The inventors also monitored changes in the latency for foot withdrawal to noxious heat in imatinib and vehicle treated mice. The inventors found that in the imatinib group the foot withdrawal latency increased to 160 ±39 % of starting values 5 days after daily imatinib treatment compared to 129 ±35% for vehicle treated mice (n=6 in each group).

### SCF produces TRPV1-dependent thermal hyperalgesia and potentiates Iₕₑₐₜ and capsaicinevoked responses in sensory neurons

This analysis indicated that c-Kit signaling is necessary to ensure normal sensing of noxious heat by polymodal nociceptors. This prompted the inventors to ask if the c-Kit ligand, SCF could acutely modulate noxious heat sensitivity in mice. The inventors monitored the latency for paw withdrawal from a radiant heat stimulus before and after the systemic administration of SCF (1 µg/g, i.p.) in awake mice, and observed a rapid and profound thermal hyperalgesia (latency drop of 37%), which became significant (t-test, p<0.05) within one hour and returned to control values after 7 hours (Figure 5A). Mice that had been pre-treated with imatinib, two subcutaneous injections (0.1 mg/g) 18 hrs and 1 hr before SCF, did not display any significant thermal hyperalgesia. Nevertheless, SCF might produce thermal hyperalgesia by an indirect mechanism, for example by enhancing the degranulation of mast cells in the skin; mast cell development requires SCF/c-Kit signaling (Besmer, 1991). Mast cells can release NGF, which might mediate the observed thermal hyperalgesia (Lewin et al., 1994). The inventors therefore used the mast cell degranulating agent 48/80 to deplete resident mast cells of neuroactive substances prior to the administration of SCF. Despite prior degranulation of mast cells, SCF injection still produced a profound hyperalgesia similar to that observed in untreated control mice (Figure 5B).

Our analysis shows that activation of the c-Kit receptor is necessary for normal noxious heat sensitivity. Furthermore, since activation of c-Kit resulted in TRPV1-dependent thermal hyperalgesia, like activation of TrkA (Galoyan et al., 2003; Shu and Mendell, 1999), c-Kit signaling might also acutely sensitize noxious heat activated currents (Cesare and McNaughton, 1996). The inventors recorded noxious heat activated currents (Iₕₑₐₜ) in mouse sensory neurons in culture before, during and after application of SCF (10 nM). Single cells were locally perfused with control Ringer's solution that could be rapidly heated to trigger noxious heat activated currents, measured at a holding potential of -60 mV (Dittert et al., 2006). Transiently applied heat stimuli typically resulted in inward currents of 200-400 pA (Figure 6A). In a single cell, repeated heating resulted in identical heat activated currents without any evidence of tachyphylaxis (interstimulus period 1-2 minutes). However, within 30 seconds after application of SCF, two populations of thermoreceptive neurons could be distinguished, one unresponsive (<50% change in heat-induced inward current concomitant with SCF stimulation) and the other showing a potentiation of the heat-dependent inward current (>50% change in heat-induced inward current concomitant with SCF application) (Figure 6A, B). The effect of SCF was short-lasting, and was not detectable 90 seconds after wash-out (Figure 6A, B). SCF did not only lead to a clear increase in the amplitude of Iₕₑₐₜ, but also produced a lowering of the heat threshold by about 2°C (Figure 6C). In responsive cells, the inventors could repeatedly induce potentiation with full recovery of Iₕₑₐₜ to control values after each washout. SCF responsive nociceptors made up 50% of the cells tested, and responsive cells tended to be those with larger initial Iₕₑₐₜ amplitudes (Figure 6B). This finding is consistent with the inventors' observation that it is primarily "high responding" C_{MH} fibers that are affected by the absence of c-Kit signaling in vivo (Figure 10C).

The inventors' data indicating that SCF can potentiate Iₕₑₐₜ suggested that the heat and pH gated ion channel TRPV1 may also be potentiated by SCF. The inventors tested this using Fura-2 based calcium imaging to quantify the increase in intracellular calcium concentration ([Ca2+]ᵢ) that follows capsaicin application to TRPV1 positive sensory neurons. The inventors used a protocol similar to that first established by McNaughton and colleagues (Bonnington and McNaughton, 2003) in which cultivated sensory neurons are exposed to two consecutive capsaicin puffs. Normally, the second [Ca2+]ᵢ transient is much smaller than the first which reflects a profound receptor desensitization that is also observed when recording capsaicin induced currents (Shu and Mendell, 1999). When the cells are exposed to NGF between the puffs a reliable sensitization is observed that is reflected in an increased [Ca2+]ᵢ transient after exposure to the second capsaicin puff (Bonnington and McNaughton, 2003; Shu and Mendell, 1999). The inventors exposed cells to a short puff (30 secs) of different concentrations of SCF between two capsaicin stimuli. In the standard protocol the inventors waited 270 secs after the SCF stimulus and then exposed the cell once again to capsaicin. With concentrations of 10 nM and 100 nM of SCF the inventors noted a very robust sensitization of the capsaicin response in ∼30% of the cells that respond to capsaicin (Fig. 6 D, E, F). The inventors' criterion for sensitization was a second capsaicin-induced [Ca2+]ᵢ signal that was larger than the mean of the second response from control experiments plus three standard deviations (second response > 120% of the first) (Fig. 6E). There were markedly fewer SCF responding cells with 0.1 nM SCF (∼7%) and the inventors observed essentially no effect with 0.001 nM SCF (Fig. 6F). The inventors also used calcium imaging to examine the speed of the SCF effect and thus used an exposure for 30 sec immediately followed by the second capsaicin stimulus (10nM short). This treatment also produced a significant sensitization of the response in 7% of the neurons tested (Fig. 6D, F), indicating that the effects of SCF can be very rapid. However, maximum effects on the TRPV1 sensitization are seen minutes after SCF stimulation. Our data are therefore consistent with the idea that SCF potentiates both Iₕₑₐₜ and the TRPV1 ion channel in TrkA positive/IB4 negative cells. Although c-Kit is restricted to a subpopulation of nociceptive neurons, treatment with its ligand SCF can produce acute thermal hyperalgesia in vivo probably by directly sensitizing a sub-population of nociceptors.

### c-Kit receptors are required for the normal development of mechanoreceptors

The studies also indicate that c-Kit is expressed in a subset of medium and large-diameter DRG mechanoreceptive sensory neurons (Figure 1). This led the inventors to ask whether the physiological properties of mechanoreceptors were altered in *c-Kit* mutant mice. Fast-conducting cutaneous afferent fibers fall into two categories: Aβ large-diameter mechanosensitive neurons (slowly-adapting mechanoreceptors, SAM, or rapidly-adapting mechanoreceptors, RAM) and slowly conducting thinly myelinated Aδ-fibers (A-mechanonociceptors, AM or D-hair afferents). These different mechanoreceptors have distinctive physiological properties that can be measured by single unit recording using the in vitro skin nerve preparation. By recording from many myelinated fibers, it is possible to determine the proportion of fibers of each receptor type (Figure 7A, Table 1). The proportion of SAM receptors is normally around 60% of the total Aβ fibers in the saphenous nerve (Koltzenburg et al., 1997; Wetzel et al., 2007), and such a proportion was also found in our control mice. In contrast, the proportion of SAM receptors was increased to >80% in *c-Kit*^{*-*/-}mice, and this was significantly different from control (p<0.01 Chi-squared test) (Figure 7A). This might reflect a selective cell death of the RAM population, or an altered differentiation of mechanoreceptors in the absence of c-Kit signaling. The inventors noted a small (∼5%) but statistically significant reduction in the number of myelinated fibers in the saphenous nerve of *c-Kit*^{*-*/*-*} mutants (Figure 3B). It is thus in principle possible that the increased number of SAM fibers encountered was entirely caused by loss of RAM fibers. The inventors noted however that the physiological properties of the SAM neurons were dramatically altered in *c-Kit*^{*-*/-}mice, suggesting that the increase in SAM number could be caused by changes in mechanoreceptor properties. SAM neurons displayed an increase in their mechanosensitivity in *c-Kit*^{*-*/*-*} compared to control mice. Thus, the number of spikes evoked by a series of displacement stimuli in *c-Kit*^{*-*/*-*} mutants was twice that of SAM fibers recorded in control mice (Figure 7B). Adaptation during a mechanical stimulus was observable in *c-Kit*^{*-*/*-*} mice, and firing rates decreased by >50% at the end of a 10 second static displacement stimulus in control and *c-Kit*^{*-*/*-*} mice (Figure 7C). The inventors presumed that the changes in the relative number of SAM neurons together with their hypersensitivity to mechanical stimuli might reflect a developmental role for the c-Kit receptor. The inventors therefore also recorded from Aβ mechanoreceptors in animals that had been treated as adults with imatinib and found no change in proportion of RAM and SAM receptors (Supplementary Figure 5). In contrast to the phenotype the inventors have observed in C_{MH} fibers they found no indication that *c-Kit* haploinsufficiency produces changes in mechanoreceptors (Figure 12). In addition no difference in the stimulus response functions of SAMs recorded in imatinib treated animals (n=20 units) compared to SAMs recorded in control mice (n=45 units) was found. Together, these data indicated that c-Kit signaling determines the normal functional properties of a sub-population of cutaneous mechanoreceptors. However, acute injection of the SCF did not change the behavioral response of mice to punctate mechanical stimulation (Figure 13).

Detailed breakdown of the single fiber recording data obtained from c-Kit +/+ and -/- mice using in vitro skin nerve preparation. CV stands for conduction velocity, vFT stands for von Frey threshold (median values are shown with the 1**^{st}** and 3**^{rd}** quartile range). Means are shown ± s.e.m. Chi**²** test, unpaired t test and Mann-Whitney test were used as statistical tests for relative percentage change, CV and vFT, respectively. ***, **, * stands for p value smaller than 0.001, 0.01, or 0.05, respectively.

**Table 1**

| | ***control*** | | | ***c-Kit-*/*-*** | | |
|---|---|---|---|---|---|---|
| **Receptor type** | % **Total** | **CV** m/s | **vFT** (mN) | **% Total** | **CV** m/s | **vFT** (mN) |
| ***A*β*-Fibers*** | | | | | | |
| RAM | 39.3 | 15.48±0.79 | 0.7 | 15** | 10.88±0.58** | 0.4 |
| | (24/61) | | (0.4-1.4) | (6/40) | | (0,4-0.85) |
| SAM | 60.7 | 15.11±0.87 | 1.4 | 85** | 11.12±0.49*** | 1*** |
| | (37/61) | | (1-2) | (34/40) | | (0.4-1) |
| ***A*δ*-Fibers*** AM | 68.6 | 5.9±0.52 | 3.6 | 54.2 | 5.43±0.42 | 2.5* |
| | (35/51) | | (2-6.3) | (32/59) | | (1.4-3.3) |
| DH | 29.5 | 4.32±0.18 | 0.4 | 45.8 | 4.55±0.22 | 0.4 |
| | (16/51) | | | (27/59) | | |
| **C-Fibers** | | | | | | |
| C- | (51) | 0.53±0.02 | 6.3 | (34) | 0.45±0.03 | 6.3 |
| units | | | (3.3-10) | | | (3.3-10) |
| C-M | 27.7 | 0.61±0.05 | 4.8 | 32 | 0.45±0.03 | 4.8 |
| | (13/47) | | (3.3-10) | (8/25) | | (2.32- |
| | | | | | | 9.07) |
| C- | 72.3 | 0.5±0.03 | 6.3 | 68 | 0.47±0.03 | 6.3 |
| MH | (34/47) | | (4.0-10) | (17/25) | | (3.3-10) |

### References

Airaksinen, M. S., and Saarma, M. (2002). The GDNF family: signalling, biological functions and therapeutic value. Nat Rev Neurosci 3, 383-394.
Bennett, D. L., Koltzenburg, M., Priestley, J. V., Shelton, D. L., and McMahon, S. B. (1998). Endogenous nerve growth factor regulates the sensitivity of nociceptors in the adult rat. Eur J Neurosci 10, 1282-1291.
Besmer, P. (1991). The kit ligand encoded at the murine Steel locus: a pleiotropic growth and differentiation factor. Curr Opin Cell Biol 3, 939-946.
Bonnington, J. K., and McNaughton, P. A. (2003). Signalling pathways involved in the sensitisation of mouse nociceptive neurones by nerve growth factor. J Physiol 551, 433-446.
Britsch, S., Li, L., Kirchhoff, S., Theuring, F., Brinkmann, V., Birchmeier, C., and Riethmacher, D. (1998). The ErbB2 and ErbB3 receptors and their ligand, neuregulin-1, are essential for development of the sympathetic nervous system. Genes Dev 12, 1825-1836.
Campana, W. M., and Myers, R. R. (2003). Exogenous erythropoietin protects against dorsal root ganglion apoptosis and pain following peripheral nerve injury. Eur J Neurosci 18, 1497-1506.
Caterina, M. J., Leffler, A., Malmberg, A. B., Martin, W. J., Trafton, J., Petersen-Zeitz, K. R., Koltzenburg, M., Basbaum, A. I., and Julius, D. (2000). Impaired nociception and pain sensation in mice lacking the capsaicin receptor. Science 288, 306-313.
Caterina, M. J., Schumacher, M. A., Tominaga, M., Rosen, T. A., Levine, J. D., and Julius, D. (1997). The capsaicin receptor: a heat-activated ion channel in the pain pathway. Nature 389, 816-824.
Cesare, P., and McNaughton, P. (1996). A novel heat-activated current in nociceptive neurons and its sensitization by bradykinin. Proc Natl Acad Sci U S A 93, 15435-15439.
Chuang, H. H., Prescott, E. D., Kong, H., Shields, S., Jordt, S. E., Basbaum, A. I., Chao, M. V., and Julius, D. (2001). Bradykinin and nerve growth factor release the capsaicin receptor from PtdIns(4,5)P2-mediated inhibition. Nature 411, 957-962.
Crowley, C., Spencer, S. D., Nishimura, M. C., Chen, K. S., Pitts-Meek, S., Armanini, M. P., Ling, L. H., McMahon, S. B., Shelton, D. L., Levinson, A. D., and et al. (1994). Mice lacking nerve growth factor display perinatal loss of sensory and sympathetic neurons yet develop basal forebrain cholinergic neurons. Cell 76, 1001-1011.
Davis, J. B., Gray, J., Gunthorpe, M. J., Hatcher, J. P., Davey, P. T., Overend, P., Harries, M. H., Latcham, J., Clapham, C., Atkinson, K., et al. (2000). Vanilloid receptor-1 is essential for inflammatory thermal hyperalgesia. Nature 405, 183-187.
Dittert, I., Benedikt, J., Vyklicky, L., Zimmermann, K., Reeh, P. W., and Vlachova, V. (2006). Improved superfusion technique for rapid cooling or heating of cultured cells under patch-clamp conditions. J Neurosci Methods 151, 178-185.
Durham, L. M. (1911). Further experiments on the inheritance of coat colour in mice. Molecular and General Genetics 6, 93-94.
Galoyan, S. M., Petruska, J. C., and Mendell, L. M. (2003). Mechanisms of sensitization of the response of single dorsal root ganglion cells from adult rat to noxious heat. Eur J Neurosci 18, 535-541.
Garratt, A. N., Voiculescu, O., Topilko, P., Charnay, P., and Birchmeier, C. (2000). A dual role of erbB2 in myelination and in expansion of the schwann cell precursor pool. J Cell Biol 148, 1035-1046.
Goldstein, M. E., House, S. B., and Gainer, H. (1991). NF-L and peripherin immunoreactivities define distinct classes of rat sensory ganglion cells. J Neurosci Res 30, 92-104.
Hayashi, S., Kunisada, T., Ogawa, M., Yamaguchi, K., and Nishikawa, S. (1991). Exon skipping by mutation of an authentic splice site of c-kit gene in W/W mouse. Nucleic Acids Res 19, 1267-1271.
Hirata, T., Kasugai, T., Morii, E., Hirota, S., Nomura, S., Fujisawa, H., and Kitamura, Y. (1995). Characterization of c-kit-positive neurons in the dorsal root ganglion of mouse. Brain Res Dev Brain Res 85, 201-211.
Hirata, T., Morii, E., Morimoto, M., Kasugai, T., Tsujimura, T., Hirota, S., Kanakura, Y., Nomura, S., and Kitamura, Y. (1993). Stem cell factor induces outgrowth of c-kit-positive neurites and supports the survival of c-kit-positive neurons in dorsal root ganglia of mouse embryos. Development 119, 49-56.
Hu, J., and Lewin, G. R. (2006). Mechanosensitive currents in the neurites of cultured mouse sensory neurones. J Physiol 577, 815-828. Huang, E. J., and Reichardt, L. F. (2003). Trk receptors: roles in neuronal signal transduction. Annu Rev Biochem 72, 609-642.
Jordt, S. E., McKemy, D. D., and Julius, D. (2003). Lessons from peppers and peppermint: the molecular logic of thermosensation. Curr Opin Neurobiol 13, 487-492.
Kerkela, R., Grazette, L., Yacobi, R., Iliescu, C., Patten, R., Beahm, C., Walters, B., Shevtsov, S., Pesant, S., Clubb, F. J., et al. (2006). Cardiotoxicity of the cancer therapeutic agent imatinib mesylate. Nat Med 12, 908-916.
Keshet, E., Lyman, S. D., Williams, D. E., Anderson, D. M., Jenkins, N. A., Copeland, N. G., and Parada, L. F. (1991). Embryonic RNA expression patterns of the c-kit receptor and its cognate ligand suggest multiple functional roles in mouse development. Embo J 10, 2425-2435.
Koltzenburg, M., Stucky, C. L., and Lewin, G. R. (1997). Receptive properties of mouse sensory neurons innervating hairy skin. J Neurophysiol 78, 1841-1850.
Kress, M., and Guenther, S. (1999). Role of [Ca2+]i in the ATP-induced heat sensitization process of rat nociceptive neurons. J Neurophysiol 81, 2612-2619.
Kruger, L., Silverman, J. D., Mantyh, P. W., Sternini, C., and Brecha, N. C. (1989). Peripheral patterns of calcitonin-gene-related peptide general somatic sensory innervation: cutaneous and deep terminations. J Comp Neurol 280, 291-302.
Lewin, G. R., and Mendell, L. M. (1993). Nerve growth factor and nociception. Trends Neurosci 16, 353-359.
Lewin, G. R., and Mendell, L. M. (1994). Regulation of cutaneous C-fiber heat nociceptors by nerve growth factor in the developing rat. J Neurophysiol 71, 941-949.
Lewin, G. R., and Moshourab, R. (2004). Mechanosensation and pain. J Neurobiol 61, 30-44.
   Lewin, G. R., Ritter, A. M., and Mendell, L. M. (1993). Nerve growth factor-induced hyperalgesia in the neonatal and adult rat. J Neurosci 13, 2136-2148.
Lewin, G. R., Rueff, A., and Mendell, L. M. (1994). Peripheral and central mechanisms of NGF-induced hyperalgesia. Eur J Neurosci 6, 1903-1912.
Little, C. C. (1915). The Inheritance of Black-Eyed White Spotting in Mice. The American Naturalist 49, 727-740.
Lourenssen, S., Motro, B., Bernstein, A., and Diamond, J. (2000). Defects in sensory nerve numbers and growth in mutant Kit and Steel mice. Neuroreport 11, 1159-1165.
Luo, W., Wickramasinghe, S. R., Savitt, J. M., Griffin, J. W., Dawson, T. M., and Ginty, D. D. (2007). A hierarchical NGF signaling cascade controls Ret-dependent and Ret-independent events during development of nonpeptidergic DRG neurons. Neuron 54, 739-754.
Malin, S. A., Molliver, D. C., Koerber, H. R., Cornuet, P., Frye, R., Albers, K. M., and Davis, B. M. (2006). Glial cell line-derived neurotrophic factor family members sensitize nociceptors in vitro and produce thermal hyperalgesia in vivo. J Neurosci 26, 8588-8599.
Motro, B., van der Kooy, D., Rossant, J., Reith, A., and Bernstein, A. (1991). Contiguous patterns of c-kit and steel expression: analysis of mutations at the W and Sl loci. Development 113, 1207-1221.
Nocka, K., Tan, J. C., Chiu, E., Chu, T. Y., Ray, P., Traktman, P., and Besmer, P. (1990). Molecular bases of dominant negative and loss of function mutations at the murine c-kit/white spotting locus: W37, Wv, W41 and W. Embo J 9, 1805-1813.
Obreja, O., Biasio, W., Andratsch, M., Lips, K. S., Rathee, P. K., Ludwig, A., Rose-John, S., and Kress, M. (2005). Fast modulation of heat-activated ionic current by proinflammatory interleukin 6 in rat sensory neurons. Brain 128, 1634-1641.
Petty, B. G., Cornblath, D. R., Adornato, B. T., Chaudhry, V., Flexner, C., Wachsman, M., Sinicropi, D., Burton, L. E., and Peroutka, S. J. (1994). The effect of systemically administered recombinant human nerve growth factor in healthy human subjects. Ann Neurol 36, 244-246.
Ramer, M. S., Bradbury, E. J., and McMahon, S. B. (2001). Nerve growth factor induces P2X(3) expression in sensory neurons. J Neurochem 77, 864-875.
Reith, A. D., Rottapel, R., Giddens, E., Brady, C., Forrester, L., and Bernstein, A. (1990). W mutant mice with mild or severe developmental defects contain distinct point mutations in the kinase domain of the c-kit receptor. Genes Dev 4, 390-400.
Rifaat, O. M. (1954). White spotting in mice and rabbits. Genetica 27, 286-292.
Ruschitzka, F. T., Wenger, R. H., Stallmach, T., Quaschning, T., de Wit, C., Wagner, K., Labugger, R., Kelm, M., Noll, G., Rulicke, T., et al. (2000). Nitric oxide prevents cardiovascular disease and determines survival in polyglobulic mice overexpressing erythropoietin. Proc Natl Acad Sci U S A 97, 11609-11613.
Russell, E., and Lawson, F. A. (1959). Selection and inbreeding for longevity of a lethal type. J Hered 50, 19-25.
Shu, X., and Mendell, L. M. (1999). Nerve growth factor acutely sensitizes the response of adult rat sensory neurons to capsaicin. Neurosci Lett 274, 159-162. Smeyne, R. J., Klein, R., Schnapp, A., Long, L. K., Bryant, S., Lewin, A., Lira, S. A., and Barbacid, M. (1994). Severe sensory and sympathetic neuropathies in mice carrying a disrupted Trk/NGF receptor gene. Nature 368, 246-249. Snider, W. D., and McMahon, S. B. (1998). Tackling pain at the source: new ideas about nociceptors. Neuron 20, 629-632.
Stein, A. T., Ufret-Vincenty, C. A., Hua, L., Santana, L. F., and Gordon, S. E. (2006). Phosphoinositide 3-kinase binds to TRPV1 and mediates NGF-stimulated TRPV1 trafficking to the plasma membrane. J Gen Physiol 128, 509-522.
Stucky, C. L., Rossi, J., Airaksinen, M. S., and Lewin, G. R. (2002). GFR alpha2/neurturin signalling regulates noxious heat transduction in isolectin B4-binding mouse sensory neurons. J Physiol 545, 43-50.
Tkaczyk, C., Horejsi, V., Iwaki, S., Draber, P., Samelson, L. E., Satterthwaite, A. B., Nahm, D. H., Metcalfe, D. D., and Gilfillan, A. M. (2004). NTAL phosphorylation is a pivotal link between the signaling cascades leading to human mast cell degranulation following Kit activation and Fc epsilon RI aggregation. Blood 104, 207-214.
Tominaga, M., Caterina, M. J., Malmberg, A. B., Rosen, T. A., Gilbert, H., Skinner, K., Raumann, B. E., Basbaum, A. I., and Julius, D. (1998). The cloned capsaicin receptor integrates multiple pain-producing stimuli. Neuron 21, 531-543.
Waskow, C., Paul, S., Haller, C., Gassmann, M., and Rodewald, H. R. (2002). Viable c-Kit(W/W) mutants reveal pivotal role for c-kit in the maintenance of lymphopoiesis. Immunity 17, 277-288.
Waskow, C., and Rodewald, H. R. (2002). Lymphocyte development in neonatal and adult c-Kit-deficient (c-KitW/W) mice. Adv Exp Med Biol 512, 1-10.
Wetzel, C., Hu, J., Riethmacher, D., Benckendorff, A., Harder, L., Eilers, A., Moshourab, R., Kozlenkov, A., Labuz, D., Caspani, O., et al. (2007). A stomatin-domain protein essential for touch sensation in the mouse. Nature 445, 206-209.
Wong, S., and Witte, O. N. (2004). The BCR-ABL story: bench to bedside and back. Annu Rev Immunol 22, 247-306.
Woodbury, C. J., Zwick, M., Wang, S., Lawson, J. J., Caterina, M. J., Koltzenburg, M., Albers, K. M., Koerber, H. R., and Davis, B. M. (2004). Nociceptors lacking TRPV1 and TRPV2 have normal heat responses. J Neurosci 24, 6410-6415.
Woolf, C. J., Safieh-Garabedian, B., Ma, Q. P., Crilly, P., and Winter, J. (1994). Nerve growth factor contributes to the generation of inflammatory sensory hypersensitivity. Neuroscience 62, 327-331.
Zhang, X., Huang, J., and McNaughton, P. A. (2005). NGF rapidly increases membrane expression of TRPV1 heat-gated ion channels. Embo J 24, 4211-4223.

## Claims

**1.** A method for identifying a compound that modulates the expression and/or activity of the tyrosine kinase receptor c-Kit in a cell, comprising the steps of a) contacting a cell expressing the tyrosine kinase receptor c-Kit with at least one potentially modulating compound, and b) measuring the modulation of the expression and/or activity of the tyrosine kinase receptor c-Kit in said cell.

**2.** The method according to claim 2, wherein said compound as identified is an inhibitor or activator of the expression and/or biological activity of the tyrosine kinase receptor c-Kit in said cell.

**3.** The method according to claim 1 or 2, wherein said method is performed in vivo in a non-human animal or in vitro.

**4.** The method according to any of claims 1 to 3, wherein said cell is selected from neuronal cells, cells recombinantly expressing the tyrosine kinase receptor c-Kit, and cells in non-human animals.

**5.** The method according to any of claims 1 to 4, wherein said activity of the tyrosine kinase receptor c-Kit is selected from the inhibition of the binding of stem cell factor to c-Kit, tyrosine kinase phosphorylation, and/or down-regulating pain transduction by sensory neurons of the dorsal root ganglion (DRG).

**6.** The method according to any of claims 1 to 5, wherein said identifying further comprises identifying a pharmaceutically active compound suitable for treating or preventing a disorder selected from pain, hyperalgesia, thermal hyperalgesia, and inflammatory pain.

**7.** The method according to any of claims 1 to 6, wherein said identifying further comprises identifying a pharmaceutically active compound that exhibits a dual effect as anti-cancer compound and analgesic compound.

**8.** The method according to any of claims 1 to 7, wherein said compound as identified is selected from a peptide library, a combinatory library, a cell extract, in particular a plant cell extract, a "small molecule drug", an antisense oligonucleotide, an siRNA, an mRNA or nucleic acid encoding c-Kit or a mutated form thereof, and an antibody or fragment thereof specifically recognizing the amino acid sequence of c-Kit, in particular the small molecule drug imatinib (4-[(4-Methyl-1-piperazinyl)methyl]-N-[4-methyl-3-[[4-(3-pyridinyl)-2-pyrimidinyl]amino]-phenyl]benzamide).

**9.** A method for manufacturing a pharmaceutical composition for treating or preventing pain, comprising the steps of: performing a method according to any of claims 1 to 8, and formulating said compound as identified into a pharmaceutical composition.

**10.** The method according to claim 9, wherein said pharmaceutical composition is for oral administration.

**11.** A pharmaceutical composition for treating or preventing pain, obtainable by a method according to claim 9 or 10.

**12.** A method for treating or preventing pain, comprising administering to a subject in need thereof an effective amount of a pharmaceutical composition according to claim 11.

**14.** Use of a compound as identified according to a method according to any of claims 1 to 8 or the pharmaceutical composition according to claim 11 for treating or preventing a disorder selected from pain, hyperalgesia, thermal hyperalgesia, and inflammatory pain.

**15.** Use of imatinib (4-[(4-Methyl-1-piperazinyl)methyl]-N-[4-methyl-3-[[4-(3-pyridinyl)-2-pyrimidinyl]amino]-phenyl]benzamide) for the production of a medicament for the treatment of a disorder selected from pain, hyperalgesia, thermal hyperalgesia, and inflammatory pain.
